# EUROPÄISCHE PATENTANMELDUNG

(11) **EP 3 502 696 A1**
(43) Veröffentlichungstag der Anmeldung: **26.06.2019**
(21) Anmeldenummer: 17209795.8
(22) Anmeldetag: 21.12.2017
(51) Int. Cl.: G01N 33/564

(54) **NACHWEIS VON AUTOANTIKÖRPERN ZUR DIAGNOSE VON DEGENERATIVEN ERKRANKUNGEN DES SKELETTSYSTEMS**

(71) Anmelder: Universität zu Köln, 50923 Köln (DE)
(72) Erfinder: Klatt, Andreas R., 50827 Köln (DE); Ruthard, Johannes, 50354 Hürth (DE); Ostendorf, Benedikt, 41460 Neuss (DE); Schneider, Matthias, 40545 Düsseldorf (DE); Pongratz, Georg, 93186 Pettendorf/Kneiting (DE); Hermes, Gabriele, 50935 Köln (DE)
(74) Vertreter: Lahrtz, Fritz

(57) **Zusammenfassung**

Die vorliegende Erfindung betrifft ein Verfahren zur Diagnose von Arthrose, umfassend das Nachweisen von einem Autoantikörper, der mit Arthrose assoziiert ist, ein Verfahren zur Diagnose einer degenerativen Erkrankung des Skelettsystems, umfassend das Nachweisen von einem Autoantikörper gegen Thrombospondin-4 oder COMP, ein Verfahren zur Diagnose von Arthrose, umfassend das Ausschließen des Vorliegens eines Autoantikörpers gegen Kollagen II, einen Kit zur Diagnose von Arthrose oder einer degenerativen Erkrankung des Skelettsystems, umfassend ein Nachweisagens für einen Autoantikörper, die Verwendung des Kits zur Diagnose von Arthrose bzw. einer degenerativen Erkrankung des Skelettsystems, einen Wirkstoff zur Verwendung zur Behandlung oder Vorbeugung einer autoimmun-assoziierten Arthrose und das Diagnoseverfahren zur Therapieauswahl von Subjekten.

SEQUENCE LISTING

<110> Universität zu Köln

<120> Nachweis von Autoantikörpern zur Diagnose von degenerativen
      Erkrankungen des Skelettsystems

<130> P70660EP

<160> 4

<170> PatentIn version 3.5

<210> 1
<211> 961
<212> PRT
<213> Homo sapiens

<400> 1

Met Leu Ala Pro Arg Gly Ala Ala Val Leu Leu Leu His Leu Val Leu
1               5                   10                  15

Gln Arg Trp Leu Ala Ala Gly Ala Gln Ala Thr Pro Gln Val Phe Asp
            20                  25                  30

Leu Leu Pro Ser Ser Ser Gln Arg Leu Asn Pro Gly Ala Leu Leu Pro
        35                  40                  45

Val Leu Thr Asp Pro Ala Leu Asn Asp Leu Tyr Val Ile Ser Thr Phe
    50                  55                  60

Lys Leu Gln Thr Lys Ser Ser Ala Thr Ile Phe Gly Leu Tyr Ser Ser
65                  70                  75                  80

Thr Asp Asn Ser Lys Tyr Phe Glu Phe Thr Val Met Gly Arg Leu Asn
                85                  90                  95

Lys Ala Ile Leu Arg Tyr Leu Lys Asn Asp Gly Lys Val His Leu Val
            100                 105                 110

Val Phe Asn Asn Leu Gln Leu Ala Asp Gly Arg Arg His Arg Ile Leu
        115                 120                 125

Leu Arg Leu Ser Asn Leu Gln Arg Gly Ala Gly Ser Leu Glu Leu Tyr
    130                 135                 140

Leu Asp Cys Ile Gln Val Asp Ser Val His Asn Leu Pro Arg Ala Phe
145                 150                 155                 160

Ala Gly Pro Ser Gln Lys Pro Glu Thr Ile Glu Leu Arg Thr Phe Gln
                165                 170                 175

Arg Lys Pro Gln Asp Phe Leu Glu Glu Leu Lys Leu Val Val Arg Gly
        180                 185                 190

Ser Leu Phe Gln Val Ala Ser Leu Gln Asp Cys Phe Leu Gln Gln Ser
    195                 200                 205

Glu Pro Leu Ala Ala Thr Gly Thr Gly Asp Phe Asn Arg Gln Phe Leu
    210                 215                 220

Gly Gln Met Thr Gln Leu Asn Gln Leu Leu Gly Glu Val Lys Asp Leu
225                 230                 235                 240

Leu Arg Gln Gln Val Lys Glu Thr Ser Phe Leu Arg Asn Thr Ile Ala
                245                 250                 255

Glu Cys Gln Ala Cys Gly Pro Leu Lys Phe Gln Ser Pro Thr Pro Ser
            260                 265                 270

Thr Val Val Pro Pro Ala Pro Pro Ala Pro Pro Thr Arg Pro Pro Arg
        275                 280                 285

Arg Cys Asp Ser Asn Pro Cys Phe Arg Gly Val Gln Cys Thr Asp Ser
    290                 295                 300

Arg Asp Gly Phe Gln Cys Gly Pro Cys Pro Glu Gly Tyr Thr Gly Asn
305                 310                 315                 320

Gly Ile Thr Cys Ile Asp Val Asp Glu Cys Lys Tyr His Pro Cys Tyr
                325                 330                 335

Pro Gly Val His Cys Ile Asn Leu Ser Pro Gly Phe Arg Cys Asp Ala
            340                 345                 350

Cys Pro Val Gly Phe Thr Gly Pro Met Val Gln Gly Val Gly Ile Ser
        355                 360                 365

Phe Ala Lys Ser Asn Lys Gln Val Cys Thr Asp Ile Asp Glu Cys Arg
    370                 375                 380

Asn Gly Ala Cys Val Pro Asn Ser Ile Cys Val Asn Thr Leu Gly Ser
385                 390                 395                 400

Tyr Arg Cys Gly Pro Cys Lys Pro Gly Tyr Thr Gly Asp Gln Ile Arg
                405                 410                 415

Gly Cys Lys Ala Glu Arg Asn Cys Arg Asn Pro Glu Leu Asn Pro Cys
            420                 425                 430

Ser Val Asn Ala Gln Cys Ile Glu Glu Arg Gln Gly Asp Val Thr Cys
        435                 440                 445

Val Cys Gly Val Gly Trp Ala Gly Asp Gly Tyr Ile Cys Gly Lys Asp
    450                 455                 460

Val Asp Ile Asp Ser Tyr Pro Asp Glu Glu Leu Pro Cys Ser Ala Arg
465                 470                 475                 480

Asn Cys Lys Lys Asp Asn Cys Lys Tyr Val Pro Asn Ser Gly Gln Glu
                485                 490                 495

Asp Ala Asp Arg Asp Gly Ile Gly Asp Ala Cys Asp Glu Asp Ala Asp
            500                 505                 510

Gly Asp Gly Ile Leu Asn Glu Gln Asp Asn Cys Val Leu Ile His Asn
        515                 520                 525

Val Asp Gln Arg Asn Ser Asp Lys Asp Ile Phe Gly Asp Ala Cys Asp
    530                 535                 540

Asn Cys Leu Ser Val Leu Asn Asn Asp Gln Lys Asp Thr Asp Gly Asp
545                 550                 555                 560

Gly Arg Gly Asp Ala Cys Asp Asp Asp Met Asp Gly Asp Gly Ile Lys
                565                 570                 575

Asn Ile Leu Asp Asn Cys Pro Lys Phe Pro Asn Arg Asp Gln Arg Asp
            580                 585                 590

Lys Asp Gly Asp Gly Val Gly Asp Ala Cys Asp Ser Cys Pro Asp Val
        595                 600                 605

Ser Asn Pro Asn Gln Ser Asp Val Asp Asn Asp Leu Val Gly Asp Ser
    610                 615                 620

Cys Asp Thr Asn Gln Asp Ser Asp Gly Asp Gly His Gln Asp Ser Thr
625                 630                 635                 640

Asp Asn Cys Pro Thr Val Ile Asn Ser Ala Gln Leu Asp Thr Asp Lys
                645                 650                 655

Asp Gly Ile Gly Asp Glu Cys Asp Asp Asp Asp Asp Asn Asp Gly Ile
            660                 665                 670

Pro Asp Leu Val Pro Pro Gly Pro Asp Asn Cys Arg Leu Val Pro Asn

675                 680                 685

Pro Ala Gln Glu Asp Ser Asn Ser Asp Gly Val Gly Asp Ile Cys Glu
    690                 695                 700

Ser Asp Phe Asp Gln Asp Gln Val Ile Asp Arg Ile Asp Val Cys Pro
705                 710                 715                 720

Glu Asn Ala Glu Val Thr Leu Thr Asp Phe Arg Ala Tyr Gln Thr Val
                725                 730                 735

Val Leu Asp Pro Glu Gly Asp Ala Gln Ile Asp Pro Asn Trp Val Val
            740                 745                 750

Leu Asn Gln Gly Met Glu Ile Val Gln Thr Met Asn Ser Asp Pro Gly
        755                 760                 765

Leu Ala Val Gly Tyr Thr Ala Phe Asn Gly Val Asp Phe Glu Gly Thr
    770                 775                 780

Phe His Val Asn Thr Gln Thr Asp Asp Asp Tyr Ala Gly Phe Ile Phe
785                 790                 795                 800

Gly Tyr Gln Asp Ser Ser Ser Phe Tyr Val Val Met Trp Lys Gln Thr
                805                 810                 815

Glu Gln Thr Tyr Trp Gln Ala Thr Pro Phe Arg Ala Val Ala Glu Pro
            820                 825                 830

Gly Ile Gln Leu Lys Ala Val Lys Ser Lys Thr Gly Pro Gly Glu His
        835                 840                 845

Leu Arg Asn Ser Leu Trp His Thr Gly Asp Thr Ser Asp Gln Val Arg
    850                 855                 860

Leu Leu Trp Lys Asp Ser Arg Asn Val Gly Trp Lys Asp Lys Val Ser
865                 870                 875                 880

Tyr Arg Trp Phe Leu Gln His Arg Pro Gln Val Gly Tyr Ile Arg Val
                885                 890                 895

Arg Phe Tyr Glu Gly Ser Glu Leu Val Ala Asp Ser Gly Val Thr Ile
            900                 905                 910

Asp Thr Thr Met Arg Gly Gly Arg Leu Gly Val Phe Cys Phe Ser Gln
        915                 920                 925

Glu Asn Ile Ile Trp Ser Asn Leu Lys Tyr Arg Cys Asn Asp Thr Ile
    930                 935                 940

Pro Glu Asp Phe Gln Glu Phe Gln Thr Gln Asn Phe Asp Arg Phe Asp
945                 950                 955                 960

Asn

<210>  2
<211>  757
<212>  PRT
<213>  Homo sapiens

<400>  2

<210>  3
<211>  197
<212>  PRT
<213>  Homo sapiens

<400>  3

Met Ala Lys Asn Gly Leu Val Ile Cys Ile Leu Val Ile Thr Leu Leu
1               5                   10                  15

Leu Asp Gln Thr Thr Ser His Thr Ser Arg Leu Lys Ala Arg Lys His
            20                  25                  30

Ser Lys Arg Arg Val Arg Asp Lys Asp Gly Asp Leu Lys Thr Gln Ile
        35                  40                  45

Glu Lys Leu Trp Thr Glu Val Asn Ala Leu Lys Glu Ile Gln Ala Leu
    50                  55                  60

Gln Thr Val Cys Leu Arg Gly Thr Lys Val His Lys Lys Cys Tyr Leu
65                  70                  75                  80

Ala Ser Glu Gly Leu Lys His Phe His Glu Ala Asn Glu Asp Cys Ile
                85                  90                  95

Ser Lys Gly Gly Ile Leu Val Ile Pro Arg Asn Ser Asp Glu Ile Asn
            100                 105                 110

Ala Leu Gln Asp Tyr Gly Lys Arg Ser Leu Pro Gly Val Asn Asp Phe
        115                 120                 125

Trp Leu Gly Ile Asn Asp Met Val Thr Glu Gly Lys Phe Val Asp Val
    130                 135                 140

Asn Gly Ile Ala Ile Ser Phe Leu Asn Trp Asp Arg Ala Gln Pro Asn
145                 150                 155                 160

Gly Gly Lys Arg Glu Asn Cys Val Leu Phe Ser Gln Ser Ala Gln Gly
                165                 170                 175

Lys Trp Ser Asp Glu Ala Cys Arg Ser Ser Lys Arg Tyr Ile Cys Glu
            180                 185                 190

Phe Thr Ile Pro Gln
        195

<210>  4
<211>  1487
<212>  PRT
<213>  Homo sapiens

<400>  4

## Beschreibung

Bei der Arthrose handelt es sich um die häufigste degenerative Gelenkerkrankung weltweit. Etwa zwei Drittel der Menschen über 65 Jahre sind von der Erkrankung betroffen. Die Ätiologie der Arthrose ist weitestgehend unbekannt. Zu den wichtigsten Risikofaktoren der Arthrose zählen Alter, Überbelastung und genetische Prädisposition. Derzeit existiert keine krankheitsmodifizierende, medikamentöse Therapie und grundlegende Probleme bei der Entwicklung von Medikamenten sind die unbekannte Ätiologie und die komplexen Pathomechanismen der Arthrose.

Ein weiteres Problem bei der Entwicklung eines krankheitsmodifizierenden Medikaments für die Arthrose ist das Fehlen sensitiver biochemischer Marker, mit denen der Erfolg einer Therapie geprüft werden kann. Die Diagnose der Arthrose erfolgt derzeit durch anamnestische Daten, Ausschluss anderer Gelenkerkrankungen, radiologische Untersuchungen und Athroskopie. Die bildgebenden Untersuchungen führen zu einer Belastung des Patienten, sind mit hohem Aufwand und Kosten verbunden, liefern jedoch nur eine grobe Aussage über das Erkrankungsstadium und können nur die späteren Krankheitsstadien darstellen. Eine Therapie- bzw. Verlaufskontrolle sowie Einteilung des Krankheitsstadiums ist mit den bisherigen Methoden kaum möglich. Die Identifikation und Validierung eines knorpel- oder knochenspezifischen Moleküls als biochemischen Marker der Arthrose ist bisher nicht gelungen. Somit existieren derzeit keine validen biochemischen Marker zur Diagnose der Arthrose.

Dean et al. (2014) beschreiben die Messung von Autoantikörper im präklinischen Stadium von rheumatoider Arthritis.

Stoll et al. (2011) beschreiben die Autoantikörper-Produktion bei juveniler idiopathischer Arthritis als Marker für den Krankheitsverlauf. Es wurden Autoantikörper gegen eine Vielzahl von Antigenen bestimmt, darunter auch Proteine der extrazellulären Matrix.

Jescke et al. beschreiben Thrombospondin-4 als Bestandteil von Gelenkknorpel. Die Autoren haben auch die Konzentration von Thrombospondin-4 im Serum von Patienten mit Arthrose gemessen. Die Thrombospondin-4-Konzentration ist gegenüber gesunden Kontroll-Probanden nicht signifikant verschieden und daher kein valider Biomarker.

Somit ist im Stand der Technik die Bestimmung von Autoantikörpern zur Diagnose von rheumatoider Arthritis, nicht jedoch von Arthrose bekannt. Während es sich bei den klassischen Rheumafaktoren um Antikörper (meist der Klasse IgM) handelt, welche die Fc-Region anderer Antikörper erkennen, sind in letzter Zeit vermehrt Autoantikörper in den Fokus gerückt, die gegen Antigene gerichtet sind, welche im Laufe von Degenerationsprozessen von Proteinen freigesetzt werden. Hierzu gehören, wie in Stoll et al. (2011) beschrieben, auch Komponenten der extrazellulären Matrix. Die wesentlichen Merkmale der vorliegenden Erfindung, nämlich die Bestimmung von Autoantikörper gegen Thrombospondin-4, COMP und/oder CLEC3A bei der Arthrose, sind jedoch nicht im Stand der Technik beschrieben.

Somit besteht ein Bedarf an Verfahren, die die Bestimmung von Arthrose bzw. von degenerativen Erkrankungen des Skelettsystems im Allgemeinen bei einem Subjekt erlauben. Weiterhin besteht ein Bedarf an Verfahren, die eine Beurteilung des Erkrankungsstadiums sowie eine Therapie- bzw. Verlaufskontrolle erlauben.

Die vorliegende Erfindung beschreibt Verfahren, die den Nachweis von Arthrose bei einem Individuum sowie die Beurteilung des Stadiums und Fortschritts der Arthrose erlauben. Die hier genannten Erfinder haben überraschenderweise entdeckt, dass Arthrose mit der Bildung von Autoantikörpern gegen Bestandteile von Knorpel oder Knochen und deren Degradationsprodukte assoziiert ist. Bei der Degeneration des Knorpels oder des Knochens kommt es zur kontinuierlichen Freisetzung von Matrixbestandteilen wie z. B. Thrombospondin-4 (TSP-4), COMP und CLEC3A. Da das Knorpelgewebe dem Immunsystem nur minder präsentiert wird, kann es im Verlauf der Erkrankung zur Stimulierung und Bildung von Autoantikörpern gegen Bestandteile von Knorpel oder Knochen bzw. gegen Neoepitope, die im Verlauf der Erkrankung bei der proteolytischen Degradation dieser Bestandteile entstehen, kommen. Dabei gehen die Erfinder davon aus, dass es in Abhängigkeit von der Menge der Freisetzung von Knorpelmatrixproteinen, z. B. TSP-4, COMP und/oder CLEC3A, zu einer Bildung von Autoantikörpern gegen diese Matrixproteine kommt. Die hier genannten Erfinder konnten im Serum von Arthrose-Patienten überraschenderweise anti-TSP-4-Autoantikörper nachweisen. Zudem wurden im Serum von weiteren Arthrose-Patienten autoreaktive Banden nachgewiesen, die deutlich kleiner als Volllängen-TSP-4 sind. Hierbei handelt es sich wahrscheinlich um Degradationsprodukte von TSP-4. Alternativ oder zusätzlich konnten die hier genannten Erfinder im Blut von Arthrose-Patienten überraschenderweise anti-COMP-Autoantikörper und/oder anti-CLEC3A-Autoantikörper nachweisen, wobei ein kombinierter Nachweis von anti-TSP-4-Autoantikörpern und anti-COMP-Autoantikörpern und/oder anti-CLEC3A-Autoantikörpern im Vergleich zu Einzelnachweisen der Autoantikörper zu einer erhöhten Nachweisrate von Arthrose führte. Bei gesunden Probanden konnten keine anti-TSP-4- und anti-CLEC3A-Autoantikörper nachgewiesen werden, und anti-COMP-Autoantikörper wurden mit sehr geringer Häufigkeit nachgewiesen. Darüber hinaus fanden die hier genannten Erfinder, dass bei keinem der Subjekte, bei denen Arthrose mit herkömmlichen Verfahren diagnostiziert worden war, anti-Kollagen II-Autoantikörper nachgewiesen werden konnten, während bei Subjekten, bei denen Arthrose mit herkömmlichen Verfahren nicht diagnostiziert worden war, anti-Kollagen II-Autoantikörper nachgewiesen werden konnten. Diese Befunde sind deswegen so überraschend, als bei Arthrose bisher kein Zusammenhang mit der Bildung von Autoantikörpern gefunden wurde und Autoantikörpern keine pathomechanistische Bedeutung zugemessen wurde.

Die hier genannten Erfinder konnten außerdem anti-TSP-4-Autoantikörper oder anti-COMP-Autoantikörper im Serum von Patienten mit durch herkömmliche Verfahren diagnostizierter rheumatoider Arthritis nachweisen, wobei manche Patienten extrem hohe Konzentrationen verglichen mit anderen RA-Patienten aufwiesen.

Somit können anti-TSP-4-Autoantikörper, anti-COMP-Autoantikörper oder anti-CLEC3A-Autoantikörper oder Autoantikörper gegen andere Bestanteile des Knorpels oder des Knochens sowie Degradationsprodukte oder Fragmente davon alleine oder in Kombination als biochemische Marker von Arthrose dienen. Der Nachweis von anti-TSP-4-Autoantikörpern, anti-COMP-Autoantikörpern oder anti-CLEC3A-Autoantikörpern oder Autoantikörpern gegen andere Bestandteile des Knorpels oder des Knochens oder von Degradationsprodukten davon alleine oder in Kombination sowie Autoantikörper-Titerbestimmungen in Proben eines Subjekts erlauben neben der Diagnose der Erkrankung eine Therapie- bzw. Verlaufskontrolle sowie eine Diagnose des Stadiums von Arthrose. Da anti-TSP-4-Autoantikörper oder anti-COMP-Autoantikörper auch in Seren von RA-Patienten nachgewiesen wurden, ist es bei der Diagnose von Arthrose unter Nachweis dieser Autoantikörper zunächst erforderlich, RA-Patienten auszuschließen. Erst dann kann eine Diagnose von Arthrose gemäß dem Verfahren der vorliegenden Erfindung erfolgen. Die Sensitivität des Nachweises kann noch erhöht werden, wenn das Subjekt auf das Vorhandensein von anti-Kollagen II-Autoantikörpern getestet wird. Wurde bei dem Subjekt aufgrund des Vorhandenseins von anti-TSP-4-Autoantikörpern, anti-COMP-Autoantikörpern und/oder anti-CLEC3A-Autoantikörpern oder Autoantikörpern gegen andere Bestanteile des Knorpels oder des Knochens sowie Degradationsprodukten oder Fragmenten davon alleine oder in Kombination Arthrose diagnostiziert, so kann die Abwesenheit des Nachweises von anti-Kollagen II-Autoantikörpern als weiteres Indiz gewertet werden, dass Arthrose vorliegt. Darüberhinaus kann das der Nachweis von anti-Kollagen II-Autoantikörpern in einem Subjekt als Indiz gewertet werden, dass keine Arthrose oder kein Risiko, Arthrose zu entwickeln, bei dem Subjekt vorliegt.

Die vorliegende Erfindung betrifft in einem 1. Aspekt ein Verfahren zur Diagnose von Arthrose (inklusive Osteochondrose) oder des Risikos, Arthrose zu entwickeln, bei einem Subjekt, wobei das Subjekt keine rheumatoide Arthritis aufweist, umfassend das Nachweisen eines Autoantikörpers, der mit Arthrose assoziiert ist, in einer von dem Subjekt stammenden Probe.

In einer Ausführungsform davon betrifft die vorliegende Erfindung dieses Verfahren, wobei das Verfahren das Ausschließen von rheumatoider Arthritis bei dem Subjekt umfasst.

In einer Ausführungsform davon betrifft die vorliegende Erfindung dieses Verfahren, wobei dieses Verfahren weiterhin das Ausschließen des Vorliegens eines Autoantikörpers gegen Kollagen II oder ein Degradationsprodukt oder ein Fragment davon umfasst, bevorzugt wobei dieses Verfahren das Nachweisen eines Autoantikörpers gegen Kollagen II oder ein Degradationsprodukt oder ein Fragment davon umfasst, stärker bevorzugt wobei dieses Verfahren das Nachweisen eines Autoantikörpers gegen Kollagen II oder ein Degradationsprodukt oder ein Fragment davon mit einem Nachweisagens zum Nachweisen eines Autoantikörpers gegen Kollagen II oder ein Degradationsprodukt oder ein Fragment davon umfasst.

In einer Ausführungsform davon betrifft die vorliegende Erfindung dieses Verfahren, wobei dieses Verfahren das Nachweisen von einem Autoantikörper gegen Thrombospondin-4 (TSP-4) oder ein Degradationsprodukt oder ein Fragment davon oder das Nachweisen von einem Autoantikörper gegen COMP oder ein Degradationsprodukt oder ein Fragment davon umfasst.

In einer Ausführungsform davon betrifft die vorliegende Erfindung dieses Verfahren, wobei der Verlauf von Arthrose überwacht wird, umfassend den Nachweis des Autoantikörpers zu verschiedenen Zeitpunkten; oder wobei das Stadium von Arthrose diagnostiziert wird.

In einer Ausführungsform davon betrifft die vorliegende Erfindung dieses Verfahren, wobei dieses Verfahren das Nachweisen von mindestens zwei Autoantikörpern umfasst, bevorzugt wobei dieses Verfahren das Nachweisen von einem Autoantikörper gegen Thrombospondin-4 (TSP-4) oder ein Degradationsprodukt oder ein Fragment davon und das Nachweisen von einem Autoantikörper gegen COMP oder ein Degradationsprodukt oder ein Fragment davon umfasst; oder bevorzugt wobei dieses Verfahren das Nachweisen von einem Autoantikörper gegen Thrombospondin-4 (TSP-4) oder ein Degradationsprodukt oder ein Fragment davon und das Nachweisen von einem Autoantikörper gegen CLEC3A oder ein Degradationsprodukt oder ein Fragment davon umfasst; stärker bevorzugt wobei dieses Verfahren das Nachweisen von einem Autoantikörper gegen Thrombospondin-4 (TSP-4) oder ein Degradationsprodukt oder ein Fragment davon, das Nachweisen von einem Autoantikörper gegen COMP oder ein Degradationsprodukt oder ein Fragment davon und das Nachweisen von einem Autoantikörper gegen CLEC3A oder ein Degradationsprodukt oder ein Fragment davon umfasst.

In einer Ausführungsform davon betrifft die vorliegende Erfindung dieses Verfahren, wobei der Autoantikörper gegen ein Neoepitop eines Degradationsprodukts eines Proteins, gegen das der Autoantikörper gerichtet ist.

In einer Ausführungsform davon betrifft die vorliegende Erfindung dieses Verfahren, wobei das Nachweisen mit einem Nachweisagens erfolgt, bevorzugt wobei das Nachweisagens fähig ist, an die Antigenbindungsregion des Autoantikörpers zu binden, stärker bevorzugt wobei das Nachweisagens TSP-4 oder ein Degradationsprodukt oder ein Fragment davon, COMP oder ein Degradationsprodukt oder ein Fragment davon und/oder CLEC3A oder ein Degradationsprodukt oder ein Fragment davon ist.

In einer Ausführungsform davon betrifft die vorliegende Erfindung dieses Verfahren, wobei die Probe Körperflüssigkeit, bevorzugt Blut, Serum, Blutplasma, Synovialflüssigkeit oder Urin, Muskel- oder Knorpelgewebe, Synovialmembran oder Sehne ist.

Der Begriff "umfasst" "umfassen", "umfassend" etc., wie hierin verwendet, bedeutet das Einschließen der offenbarten Merkmale und weiterer Merkmale, die nicht spezifisch erwähnt sind. Der Begriff "umfasst" "umfassen", "umfassend" etc. wird auch in dem Sinn von "bestehend aus" den offenbarten Merkmalen verstanden, wobei weitere Merkmale als die offenbarten nicht eingeschlossen sind. So kann das Verfahren der vorliegenden Erfindung weitere Verfahrensschritte umfassen, kann aber auch nur aus den spezifizierten Schritten bestehen. Auch der Kit der vorliegenden Erfindung kann weitere Bestandteile umfassen, kann aber auch nur aus den spezifizierten Bestandteilen bestehen.

Thrombospondin-4 ist ein Protein, das in Menschen von dem TSP-4-Gen codiert wird. Das Protein gehört zur Thrombospondin-Proteinfamilie. Thrombospondin-Familienmitglieder sind extrazelluläre, adhäsive glykosylierte Matrixproteine, die verbreitet in Vertebraten exprimiert werden (Lawler et al., 1995). Es sind fünf Thrombospondin-Proteine, Thrombospondin-1 bis Thrombospondin-4 und oligomeres Knorpelmatrix-Protein (COMP), bekannt. Die Mitglieder der Thrombospondin-Familie werden während Zellumbauprozessen aktiviert und vermitteln Zell-zu-Zell- und Zell-zu-Matrix-Interaktionen. Thrombospondin-4, eine pentameres Protein, spielt bei zellulärer Migration, Adhäsion sowie Proliferation eine Rolle, wobei es an Heparin und Calcium binden kann. Es wird unter anderem in Muskel- und Knochengewebe gebildet.

TSP-4 hat eine Länge von 961 Aminosäuren, wobei die Aminosäuren 1 bis 26 das Signalpeptid und die Aminosäuren 27 bis 961 das reife Protein bilden. Für veranschaulichende Zwecke, ohne darauf beschränkt zu sein, wird auf die Aminosäuresequenz von Thrombospondin-4 Bezug genommen, die hierin als SEQ ID NR: 1 offenbart ist. Die entsprechende Nukleotid- und Aminosäuresequenzen sind von der NCBI (National Centre for Biotechnology Information; National Library of Medicine, Bethesda, MD20894, USA; www.ncbi.nlm.nih.gov) unter der Zugangsnummer UniProtKB - P35443.2 (SEQ ID NR: 1) erhältlich. 
1 MLAPRGAAVL LLHLVLQRWL AAGAQATPQV FDLLPSSSQR LNPGALLPVL TDPALNDLYV
61 ISTFKLQTKS SATIFGLYSS TDNSKYFEFT VMGRLNKAIL RYLKNDGKVH LVVFNNLQLA
121 DGRRHRILLR LSNLQRGAGS LELYLDCIQV DSVHNLPRAF AGPSQKPETI ELRTFQRKPQ
 
181 DFLEELKLVV RGSLFQVASL QDCFLQQSEP LAATGTGDFN RQFLGQMTQL NQLLGEVKDL
241 LRQQVKETSF LRNTIAECQA CGPLKFQSPT PSTVVPPAPP APPTRPPRRC DSNPCFRGVQ
301 CTDSRDGFQC GPCPEGYTGN GITCIDVDEC KYHPCYPGVH CINLSPGFRC DACPVGFTGP
361 MVQGVGISFA KSNKQVCTDI DECRNGACVP NSICVNTLGS YRCGPCKPGY TGDQIRGCKA
421 ERNCRNPELN PCSVNAQCIE ERQGDVTCVC GVGWAGDGYI CGKDVDIDSY PDEELPCSAR
481 NCKKDNCKYV PNSGQEDADR DGIGDACDED ADGDGILNEQ DNCVLIHNVD QRNSDKDIFG
541 DACDNCLSVL NNDQKDTDGD GRGDACDDDM DGDGIKNILD NCPKFPNRDQ RDKDGDGVGD
601 ACDSCPDVSN PNQSDVDNDL VGDSCDTNQD SDGDGHQDST DNCPTVINSA QLDTDKDGIG
661 DECDDDDDND GIPDLVPPGP DNCRLVPNPA QEDSNSDGVG DICESDFDQD QVIDRIDVCP
721 ENAEVTLTDF RAYQTVVLDP EGDAQIDPNW VVLNQGMEIV QTMNSDPGLA VGYTAFNGVD
781 FEGTFHVNTQ TDDDYAGFIF GYQDSSSFYV VMWKQTEQTY WQATPFRAVA EPGIQLKAVK
841 SKTGPGEHLR NSLWHTGDTS DQVRLLWKDS RNVGWKDKVS YRWFLQHRPQ VGYIRVRFYE
901 GSELVADSGV TIDTTMRGGR LGVFCFSQEN IIWSNLKYRC NDTIPEDFQE FQTQNFDRFD
961 N
 (SEQ ID NR: 1)

Ein anderes Protein der Thrombospondin-Proteinfamilie ist das oligomere Knorpelmatrix-Protein (COMP), auch als Thrombospondin-5 bekannt. Es ist ein extrazelluläres Matrix (ECM)-Protein, das hauptsächlich im Knorpel vorkommt. Spezifisch kommt COMP in der extrazellulären Matrix, die Zellen umgibt, die Bänder und Sehnen ausmachen, und nahe Knorpel-bildenden Zellen (Chondrozyten) vor. Im Menschen wird das COMP-Protein von dem *COMP*-Gen codiert. Bindung an andere ECM-Proteins wie Kollagen scheint von divalenten Kationen abzuhängen. COMP ist ein Marker für Knorpel-Turnover. Es scheint eine Rolle bei der vaskulären Wandummodellierung zu haben. Darüber hinaus scheint es eine Rolle bei Zellwachstum und Zellteilung und der Apoptose sowie der Regulierung von Zellbewegung und Zellanheftung zu spielen. Es kann die Interaktion von Chondrozyten mit extrazellulärer Knorpelmatrix durch Interaktion mit Zelloberflächen-Integrinrezeptoren vermitteln. Das Protein besteht aus fünf identischen Glykoprotein-Untereinheiten, jede mit EGF-ähnlichen und Calcium-bindenden (Thrombospondinähnlichen) Domänen, die stark an Calcium binden (1, 2, 3, 4, 5, 6, 7, 8, 9).

COMP hat eine Länge von 757 Aminosäuren. Für veranschaulichende Zwecke, ohne darauf beschränkt zu sein, wird auf die Aminosäuresequenz von COMP Bezug genommen, die hierin als SEQ ID NR: 2 offenbart ist. Die entsprechende Nukleotid- und Aminosäuresequenzen sind von der NCBI (National Center for Biotechnology Information; National Library of Medicine, Bethesda, MD20894, USA; www.ncbi.nlm.nih.gov) unter der Zugangsnummer UniProtKB - P49747.2 (SEQ ID NR: 2) erhältlich. 
1       
61      
121     
181     
241     
301     
 
361 
421 
481 
541 
601 
661 
721 
 (SEQ ID NR: 2)

C-Typ-Lektindomänen-Familie 3-Mitglied A (CLEC3A; englisch: C-type lectin domain family 3 member A) ist ein knorpelspezifisches Protein, welches in arthrotischem Knorpel verstärkt exprimiert wird. Neben Knorpel wurde es bisher nur in Brustkrebsgewebe nachgewiesen. CLEC3A besteht aus drei Domänen: einer stark positiv geladenen N-terminalen Domäne, gefolgt von einer alpha-helikalen Oligomerisierungsdomäne und einer C-terminalen Kohlenhydrat-Erkennungsdomäne (CRD; englisch: Carbohydrate Recognition Domain). CLEC3A bindet an Laminin und Fibronektin und wird von Matrilysin (MMP-7) und anderen Matrixproteasen geschnitten (Tsunezumi, 2009). Vor kurzem wurde eine schnellere Aktivierung von Plasminogen durch tissue-Plasminogenaktivator in der Gegenwart von CLEC3A gezeigt (10, 11, 12, 13).

CLEC3A hat eine Länge von 197 Aminosäuren, wobei die Aminosäuren 1 bis 22 das Signalpeptid und die Aminosäuren 23 bis 197 das reife Protein bilden. Für veranschaulichende Zwecke, ohne darauf beschränkt zu sein, wird auf die Aminosäuresequenz von CLEC3A Bezug genommen, die hierin als SEQ ID NR: 3 offenbart ist. Die entsprechende Nukleotid- und Aminosäuresequenzen sind von der NCBI (National Centre for Biotechnology Information; National Library of Medicine, Bethesda, MD20894, USA; www.ncbi.nlm.nih.gov) unter der Zugangsnummer UniProtKB - O75596.1 SEQ ID NR: 3) erhältlich. 
1       MAKNGLVICI LVITLLLDQT TSHTSRLKAR KHSKRRVRDK DGDLKTQIEK LWTEVNALKE
61      IQALQTVCLR GTKVHKKCYL ASEGLKHFHE ANEDCISKGG ILVIPRNSDE INALQDYGKR
121     SLPGVNDFWL GINDMVTEGK FVDVNGIAIS FLNWDRAQPN GGKRENCVLF SQSAQGKWSD
181     EACRSSKRYI CEFTIPQ
 (SEQ ID NR: 3)

Kollagen (Vorstufe Tropokollagen) ist ein nur bei vielzelligen Tieren (einschließlich Menschen) vorkommendes Strukturprotein hauptsächlich des Bindegewebes, genauer der extrazellulären Matrix). Kollagen besteht aus einzelnen, langen Kollagenmolekülen, die eine linksgängige Helix ausbilden. Jeweils drei dieser Helices sind in einer rechtsgängigen Superhelix arrangiert. Die Tripelhelix wird durch Wasserstoffbrücken zwischen den einzelnen Strängen stabilisiert. Kollagen Typ II, das in der vorliegenden Anmeldung für die Diagnose von Arthrose oder des Risikos, Arthrose zu entwickeln, eine Rolle spielt, kommt hauptsächlich im Knorpel vor und fungiert als Strukturprotein des hyalinen und des elastischen Knorpels. Die Gewinnung von Kollagen II erfolgt durch Isolierung aus Knorpel nach dem Verfahren von Vogel und Paulsson (1984) (14). Die entsprechende Nukleotid- und Aminosäuresequenzen sind von der NCBI (National Centre for Biotechnology Information; National Library of Medicine, Bethesda, MD20894, USA; www.ncbi.nlm.nih.gov) unter der Zugangsnummer UniProtKB - P02458.3 (SEQ ID NR: 4) erhältlich.

1 
61 
121 
181 
241 
301 
361 
421 
481 
541 
601 
661 
721 
781 
841 
901 
961 
1021 
1081 
1141 
1201 
1261 
1321 
1381 
1441 

### (SEQ ID NR: 4)

Der Begriff "TSP-4", "COMP", "CLEC3A" oder "Kollagen II", wie hierin verwendet, ist jegliches Protein, das als TSP-4, COMP, CLEC3A bzw. Kollagen II bekannt ist. Der Begriff umfasst auch ein Protein, das gleiche oder ähnliche Funktionen wie TSP-4, COMP, CLEC3A bzw. Kollagen II, identifiziert durch SEQ ID NR: 1, SEQ ID NR: 2, SEQ ID NR: 3 und SEQ ID NR: 4, aufweist. Dabei können die Proteine auch unter einem anderen Namen bekannt sein. Alternativ bezieht sich der Begriff TSP-4, COMP, CLEC3A bzw. Kollagen II auf jedes Protein, das eine Aminosäureidentität zu der Sequenz von SEQ ID NR: 1, SEQ ID NR: 2, SEQ ID NR: 3 und SEQ ID NR: 4 von mindestens 50%, 60%, 70%, 80%, 85%, 90%, 95%, 96%, 97%, 98%, oder 99% hat. Alternativ bezieht sich der Begriff TSP-4, COMP, CLEC3A bzw. Kollagen II auf jedes Protein, das eine Aminosäurehomologie zu der Sequenz von SEQ ID NR: 1, SEQ ID NR: 2, SEQ ID NR: 3 und SEQ ID NR: 4 von mindestens 50%, 60%, 70%, 80%, 85%, 90%, 95% oder 100% hat. Funktionen bzw. Eigenschaften von TSP-4, COMP, CLEC3A bzw. Kollagen II sind: TSP-4 bildet Pentamere und bindet an Heparin. COMP bildet auch Pentamere, bindet an Kollagen II und ist offenbar an der Fibrillogenese von Kollagen II beteiligt. Mutationen im COMP-Gen können PSACH (osteochondrodysplasias pseudoachondroplasia) und MED (multiple epiphyseal dysplasia) verursachen. CLEC3A führt zu einer schnelleren Aktivierung von Plasminogen durch tissue-Plasminogenaktivator. Kollagen II ist Strukturbestandteil der extrazellulären Knorpelmatrix, bildet das Knorpelgrundgerüst und gibt dem Knorpel seine Zugfestigkeit. Unter den Begriff TSP-4, COMP, CLEC3A bzw. Kollagen II fallen jegliche Proteine, an die Autoantikörper, die in Subjekten gegen TSP-4, COMP, CLEC3A bzw. Kollagen II gebildet werden, binden.

Der Begriff "TSP-4", "COMP" , "CLEC3A" oder "Kollagen II", wie hierin verwendet, bezieht sich auch auf ein Fragment von TSP-4, COMP, CLEC3A bzw. Kollagen II, solange dieses Fragment die Fähigkeit besitzt, an Autoantikörper, die in Subjekten gegen TSP-4, COMP, CLEC3A bzw. Kollagen II gebildet werden, zu binden. Bevorzugt enthalten solche Fragmente Epitope, gegen die die Autoantikörper gerichtet sind. Stärker bevorzugt haben Fragmente von TSP-4, COMP, CLEC3A bzw. Kollagen II eine Länge von mindestens 5 Aminosäuren, noch stärker bevorzugt eine Länge von 5 bis 100, noch stärker bevorzugt eine Länge von 5 bis 50, noch stärker bevorzugt eine Länge von 10 bis 50, noch stärker bevorzugt eine Länge von 15 bis 50 noch stärker bevorzugt eine Länge von 15 bis 25 oder 40 bis 50, Aminosäuren.

Der Prozentsatz der "Aminosäureidentität", wie hierin verwendet, bezieht sich auf den Prozentsatz von Aminosäureresten, die an entsprechenden Positionen in zwei optimal zueinander ausgerichteten Sequenzen identisch sind. Er wird durch Vergleich von zwei optimal zueinander ausgerichteten Sequenzen über ein Vergleichsfenster, wobei das Fragment der Aminosäuresequenz in dem Vergleichsfenster Additionen oder Deletionen (z. B. Lücken oder Überhänge) umfassen kann, im Vergleich zu der Referenzsequenz SEQ ID NR: 1, SEQ ID NR: 2, SEQ ID NR: 3 und SEQ ID NR: 4, die diese Additionen oder Deletionen nicht enthält, zur optimalen Ausrichtung der zwei Sequenzen bestimmt. Der Prozentsatz wird berechnet durch Bestimmen der Anzahl von Positionen, an denen der identische Aminosäurerest in beiden Sequenzen vorkommt, um die Anzahl an gleichen Positionen zu erhalten, Teilen der Anzahl der gleichen Positionen durch die Gesamtzahl der Positionen in dem Vergleichsfenster und Multiplizieren des Ergebnisses mit 100. Optimale Ausrichtung der Sequenzen für den Vergleich kann durch den Homologiealgorithmus von Smith und Waterman, 1981, durch den Homologie-Alignment-Algorithmus von Needleman und Wunsch, 1970, durch die Suche nach Ähnlichkeitsverfahren von Pearson und Lipman, 1988, durch den Algorithmus von Karlin und Altschul, 1990, modifiziert durch Karlin und Altschul, 1993, oder durch Computerimplementationen dieser Algorithmen (GAP, BESTFIT, BLAST, PASTA und TFASTA in dem Wisconsin Genetics Software-Packet, Genetics Computer Group (GCG), 575 Science Dr., Madison, WI) durchgeführt werden. GAP und BESTFIT werden bevorzugt verwendet, um die optimale Ausrichtung zu bestimmen. Typischerweise werden die Standardeinstellungswerte von 5,00 für "gap weight" und 0,30 für "gap weight"-Länge verwendet.

Der Begriff "Prozent Homologie", wie hierin verwendet, bezieht sich auf den Prozentsatz von Aminosäureresten, die an entsprechenden Positionen in zwei optimal zueinander ausgerichteten Sequenzen homolog sind. Der "Prozent Homologie" zwischen zwei Sequenzen ist in einer Weise etabliert, die dem, was vorstehend in Bezug auf die Bestimmung des "Prozentsatzes an Identität" beschrieben wurde, im Wesentlichen identisch ist, außer der Tatsache, dass bei der Berechnung auch homologe Positionen und nicht nur identische Positionen berücksichtigt werden. Zwei homologe Aminosäuren sind entweder zwei identische oder zwei homologe Amoinosäuren. Homologe Aminosäurereste haben ähnliche chemisch-physikalische Eigenschaften, zum Beispiel Aminosäuren, die zu der gleichen Gruppe gehören: aromatisch (Phe, Trp, Tyr), sauer (Glu, Asp), polar (Gln, Asn), basisch (Lys, Arg, His), aliphatisch (Ala, Leu, lie, Val), mit einer Hydroxylgruppe (Ser, Thr) oder mit einer kurzen Seitenkette (Gly, Ala, Ser, Thr, Met). Es wird erwartet, dass Substitutionen zwischen solchen homologen Aminosäuren den Proteinphänotyp nicht verändern (konservative Substitutionen).

Der Begriff "Arthrose" bezeichnet eine nicht-entzündliche, mit einer Zustandsänderung verbundene Gelenkerkrankung, kurz Gelenkverschleiß oder Gelenkabnutzung. Dabei wird der Gelenkknorpel meistens über einen längeren Zeitraum chronisch geschädigt, was Arthrose als degenerative Erkrankung ausweist. Die Erkrankung hinterlässt mit der Zeit auch im benachbarten Gewebe, der Gelenkkapsel, dem Knochen und der Muskulatur Spuren. Oft werden auch die Ausdrücke Arthrosis deformans, Osteoarthrose, degenerative Arthropathie oder Osteoarthritis verwendet, die das gleiche Krankheitsbild beschreiben. Dabei weist die Vorsilbe "Osteo" darauf hin, dass auch der Gelenkknochen an der Krankheit beteiligt ist. Als Ursache werden ein Übermaß an Belastung (etwa erhöhtes Körpergewicht), angeborene oder traumatisch bedingte Ursachen wie Fehlstellungen der Gelenke oder auch knöcherne Deformierung durch Knochenerkrankungen wie Osteoporose gesehen. Grundsätzlich können alle Gelenke von arthrotischen Veränderungen betroffen sein. Man unterscheidet mehrere Arten von Arthrose. Bei der primären Arthrose wird eine biologische Minderwertigkeit des Knorpelgewebes unklarer Ursache angenommen. Sekundäre Arthrosen entstehen durch mechanische Überlastung (etwa bei Hüftgelenksdysplasie), entzündliche Veränderungen (etwa bei Arthritiden) oder metabolische Störungen (etwa bei Chondrokalzinose). Die Arthrose kann auch mit überlastungsbedingter Ergussbildung (sekundäre Entzündungsreaktion) einhergehen (aktivierte Arthrose). In dem Begriff "Arthrose" ist Osteochondrose eingeschlossen.

Die Arthrose wird nach dem Röntgenbefund in die Kellgren-Lawrence-Scores 1 bis 4 oder nach dem Ausmaß der Knorpelschädigung in die Outerbridge-Klassifikationen I bis IV eingeteilt. Bei der Arthrose führt eine anfängliche Knorpelschädigung im weiteren Verlauf zu Veränderungen am Knochen. Im Stadium I der Outerbridge-Klassifikation kommt es zu Rauigkeiten und Ausdünnung der Knorpelschicht, tangentiale Fissuren treten auf. Im Stadium II wird hyaliner Knorpel durch Granulationsgewebe und minderwertigere Faserknorpel ersetzt. Es bilden sich Pseudozysten aus nekrotischem Knorpel- und Knochengewebe (Geröllzyste). Im Stadium III treten bereits Ulcerationen auf, das Bindegewebe und die Chondrozyten proliferieren. Im Stadium IV flacht die Knochenplatte eines Gelenkes ab. Um den Druck auf dem Gelenk dennoch abzufangen, bilden sich Randwülste am Knochen (Osteophyten).

**Tabelle 1: Übersicht über röntgenologische Veränderungen zur Bestimmung der Kellgren-Lawrence-Scores. Die jeweils zutreffende Zeile bestimmt den Grad.**

| Grad | Röntgenbefunde | | |
|---|---|---|---|
| | Subchondrale Sklerose | Gelenkspaltverschmälerung | Osteophyten-Bildung |
| 1 | gering | nicht vorhanden | nicht vorhanden |
| 2 | unregelmäßige Gelenkfläche angedeutet | gering | gering |
| 3 | unregelmäßige Gelenkfläche deutlich erkennbar | deutlich | deutlich |
| 4 | ausgeprägte Gelenkveränderungen bis zur vollständigen Destruktion. Sichtbare Deformierung/Nekrotisierung der Gelenkpartner | | |

Die Diagnose der Arthrose erfolgt herkömmlicherweise abhängig vom Gelenkbefall (Hand, Hüfte, Knie) unterschiedlich. Hauptkriterium ist der Gelenkschmerz (Belastungs-, Anlauf-, Ermüdungs-, Dauer-, Nacht-, Endphasenschmerz und Schmerzausstrahlung). Weitere Kriterien sind typische Röntgenbefunde (siehe Kellgren-Lawrence-Score), Bewegungseinschränkungen (z.B. Einschränkung der Innenrotation auf unter 15° in der Hüfte), kurze Phase der Morgensteifigkeit (< 30 min Knie, < 60 min Hüfte), Gelenkvergrößerung/Veränderung ohne weitere klinischen Entzündungszeichen, Krepitationen unter Palpation der befallenen Gelenke, höheres Alter (> 50 Jahre), BSG < 40 mm/h, normales CRP (< 5 mg/l), negativer RF und Anti-CCP. Die Diagnose Arthrose darf gestellt werden, wenn das Hauptkriterium und weitere klinische oder serologische Kriterien erfüllt sind (für Details siehe: https://www.hopkinsarthritis.org/physician-corner/education/arthritis-educationdiagnostic-guidelines/(15-17)).

Die "rheumatoide Arthritis" (RA) ist die häufigste entzündliche Erkrankung der Gelenke. Sie kommt bei ca. 1 % der erwachsenen Bevölkerung vor. Der Krankheitsbeginn ist oft schleichend und geht mit Schmerzen in den kleinen Finger- oder Zehengelenken einher, wobei aber auch andere Gelenke betroffen sein können, insbesondere Hand-, Knie-, Schulter, Fuß- und Hüftgelenke. Typischerweise werden bevorzugt die Handwurzelknochen, die Fingergrundgelenke und die proximalen Interphalangealgelenke befallen. Die betroffenen Gelenke schwellen an und sind überwärmt. Eine Rötung der betroffenen Gelenke kann hinzukommen. Morgens sind diese Symptome meist am stärksten ausgeprägt (Morgensteifigkeit). Im Krankheitsverlauf werden immer mehr Gelenke befallen. Meist verläuft die Krankheit schubweise; ein Schub dauert typischerweise zwischen einigen Wochen und Monaten an. Zwischen den einzelnen Schüben lassen die Beschwerden nach.

Die Diagnose von RA ist relativ sicher und erfolgt durch Labordiagnostik, Klinikdiagnostik und bildgebende Verfahren entsprechend der ACR/EULAR-Klassifikationskriterien (Aletaha et al., 2010)

**Tabelle 2: Diagnoseschema nach den ACR/EULAR-Kriterien. Pro Spalte wird der höchste erreichte Punktwert vergeben. Die Spaltenpunkte werden summiert. Eine Rheumatoide Arthritis ist gesichert bei ≥6 Punkte und gesicherter Synovitis in einem typischen Gelenk (sofern für das entzündete Gelenk keine andere Ursache für die Synovitis vorliegt). RF=Rheuma Faktor, Anti-CCP-AK=Antikörper gegen cyclischcitrullinierte Peptide, CRP=C-Reaktives Protein, BSG=Blutsenkungsgeschwindigkeit.**

| **Punkte** | **geschwollene /schmerzhafte Gelenke** | **Serologie** | **Entzündungsparameter im Blut** | **Symptomdauer*** |
|---|---|---|---|---|
| **0** | ≤1 großes Gelenk*** | RF + Anti-CCP-AK negativ | CRP + BSG normal | <6 Wochen |
| **1** | 2-10 große Gelenke*** | | CRP oder BSG↑ | ≥6 Wochen |
| **2** | 1-3 kleine Gelenke** | RF oder Anti-CCP-AK erhöht | | |
| **3** | 4-10 kleine Gelenke** | RF oder Anti-CCP-AK stark erhöht (>3-fach über Referenzwert) | | |
| **5** | >10 Gelenke & ≥1 kleines Gelenk** | | | |
| * Des am längsten betroffenen Gelenkes, | | | | |
| ** Fingergrund-(MCP) und Fingermittelgelenke (PIP) I-V; Zehengrundgelenke (MTP) II-V, Großzehenmittelgelenke und Handgelenke. Ausgeschlossen sind: Daumensattelgelenke, Großzehengrundgelenke (MTP I), Finger- und Zehenendgelenke (DIP), | | | | |
| *** Sprung-, Knie-, Hüft-, Ellenbogen- und Schultergelenk | | | | |

Bei den bildgebenden Verfahren werden Röntgen- oder MRT-Untersuchungen verwendet, um Schädigungen der Knochen (Erosionen) abschätzen zu können. Typische radiologische Befunde sind subchondrale Osteoporose, Destruktionen des umliegenden Knochens, Ankylosen und Gelenkfehlstellungen (Knopflochdeformität, Schwanenhalsdeformität, Ulnardeviation). Mit der Weichteil- und Knochenszintigraphie kann das Verteilungsmuster der Entzündungsaktivität der verschiedenen Gelenke recht gut dargestellt werden.

Um gemäß dem Verfahren der vorliegenden Erfindung Arthrose oder das Risiko, Arthrose zu entwickeln, bei einem Subjekt zu diagnostizieren, muss RA bei diesem Subjekt ausgeschlossen werden. Dies erfolgt unter Verwendung von im Fachgebiet bekannten Verfahren. Der Begriff "wobei das Subjekt keine rheumatoide Arthritis aufweist" bedeutet deshalb, dass das Subjekt mit den gängigen Methoden auf RA untersucht wird. Wenn mit diesen Methoden RA nicht festgestellt wird, dann weist das Subjekt im Sinne der Erfindung keine RA auf und kann dem Verfahren der vorliegenden Erfindung zur Diagnose von Arthrose oder zur Diagnose des Risikos, Arthrose zu entwickeln, zugeführt werden. Der Ausschluss von RA kann vor, während oder nach dem Nachweis von Autoantikörpern, die mit Arthrose assoziiert sind, erfolgen, erfolgt bevorzugt aber, bevor die Diagnose von Arthrose oder des Risikos, Arthrose zu entwickeln, durchgeführt wird.

Autoantikörper sind Antikörper, die von einem Subjekt in Reaktion auf ein natives Protein (Autoantigen) erzeugt werden. In der Regel erzeugen Individuen keine Immunantwort auf native Proteine und produzieren deshalb keine Antikörper gegen sie. Doch in seltenen Fällen werden die eigenen nativen Proteine als Antigene erkannt, woraufhin B-Zellen, die solche Autoantikörper bilden, stimuliert und Autoantikörper produziert werden. Dies kann zu verschiedenen Autoimmunerkrankungen führen. Die geringe Präsentation von Knochen- und Knorpelgewebe gegenüber dem Immunsystem kann der Grund für die Bildung von Autoantikörpern gegen Bestandteile von Knorpel oder Knochen bzw. gegen Neoepitope, die bei der proteolytischen Degradation dieser Bestandteile neu entstehen, sein. In der vorliegenden Erfindung wird der Nachweis von Autoantikörper gegen extrazelluläre Matrixproteine, deren Degradationsprodukte oder Fragmente davon zur Diagnose von Arthrose oder einer degenerativen Erkrankung des Skelettsystems verwendet.

Der Begriff "Autoantikörper, der mit Arthrose assoziiert ist", wie hierin verwendet, bezieht sich auf Autoantikörper in Subjekten, die an einer Arthrose leiden oder die eine Arthrose entwickeln werden, während ein solcher Autoantikörper in Subjekten ohne Arthrose oder die keine Arthrose entwickeln werden, z. B. mit den hier in Bezug genommenen Verfahren, z. B. einem Antikörper-basiertem Testverfahren, z. B. einem Immunblottest, nicht nachgewiesen werden kann. Autoantikörper, die mit Arthrose assoziiert sind, sind im Rahmen der vorliegenden Erfindung solche, die gegen Proteine der extrazellulären Knorpelmatrix, Proteine der subchondralen extrazellulären Knochenmatrix, Proteine der Gelenkkapsel oder Proteine der extrazellulären Muskel und Sehnenmatrix, folglich alle Proteine, die im Rahmen der Degradations/Enzündungsprozesse am Gelenk freigesetzt werden können, gerichtet sind. Bevorzugt sind die Autoantikörper gegen Knorpelmatrixproteine außer Kollagen II gerichtet, wobei stärker bevorzugt die Autoantikörper gegen TSP-4, COMP und/oder CLEC3A gerichtet sind. Im Tierversuch wurde nachgewiesen, dass Autoantikörper gegen Knorpelmatrixproteine wie COMP eine pathophysiologische Bedeutung haben und zu schwerer chronischer Arthritis führen können. Somit ist das Vorliegen von Autoantikörpern, die mit Arthrose assoziiert sind, oder von Autoantikörpern, die mit einer degenerativen Erkrankung des Skelettsystems assoziiert sind, bei einem Subjekt ein Hinweis auf eine pathophysiologische Rolle dieser Autoantikörper beim Entstehen, beim Aufweisen und bei der Progression von Arthrose bzw. der degenerativen Erkrankung des Skelettsystems.

Wie hierin verwendet, beinhaltet der Begriff "ein Autoantikörper" ein oder mehrere Autoantikörper. Somit umfasst der Begriff "Nachweisen eines Autoantikörpers" das Nachweisen von einem (1) spezifischen Autoantikörper mit z. B. einem (1) Nachweisagens als auch das Nachweisen von mehr als einem (1) spezifischen Autoantikörper wie 2, 3, 4, 5 oder mehr Autoantikörpern mit z. B. mehr als einem (1) Nachweisagens wie 2, 3, 4, 5 bzw. mehr Nachweisagentien.

Knorpelmatrixproteine sind Proteine, die in der Knorpelmatrix vorkommen. Als Knorpelmatrix bezeichnet man die zwischen den Knorpelzellen gelegene extrazelluläre Matrix (EZM) des Knorpelgewebes. Die Knorpelmatrix besteht aus einer unstrukturierten Grundsubstanz und einem organisierten Netz aus Kollagenfasern. Sie kann in zwei Bereiche unterteilt werden: die territoriale Matrix (Knorpelhof) und die interterritoriale Matrix (Interterritorien, Interterritorialzone). Sie besteht aus einem dichten Netzwerk von Kollagenfasern und bildet zusammen mit den Chondrozyten die Chondrone ("Territorien"). Die territoriale Matrix umgibt die Chondrozyten und schließt sie dadurch ein. Typische Proteine der Knorpelmatrix sind Kollagene (Kollagen I, II und III), Proteine der Thrombospondinfamilie und CLEC3A.

Der Begriff "Degradationsprodukt", wie hierin verwendet, bezeichnet ein Fragment eines Proteins, z.B. TSP-4, COMP oder CLEC3A, das auf natürliche Weise in einem Subjekt durch Abbauprozesse, z.B. unter Einwirkung von Proteasen, aus dem Volllängenprotein hervorgegangen ist. TSP-4, COMP und CLEC3A unterliegen dabei, wie auch andere Proteine der extrazellulären Matrix, dem Abbau durch sezernierte oder membranständige Proteasen auf zellulärer Ebene. Ein Degradationsprodukt kann ein Epitop umfassen, das bei dem nativen Protein bereits vorhanden ist und deshalb von einem Autoantikörper erkannt wird, der auch das native Protein aufgrund des Epitops erkennt. Alternativ kann ein Degradationsprodukt ein Neoepitop umfassen, das aufgrund der Degradation neu gebildet wird. Solche Neoepitope werden normalerweise nicht durch Autoantikörper gegen die nativen Volllängenproteine erkannt, sondern können die Bildung neuer Autoantikörper stimulieren.

Wie hierin verwendet, versteht man unter "Nachweisagens" ein beliebiges Molekül, eine beliebige Substanz oder ein beliebiges Reagenz, das/die spezifisch an den Autoantikörper bindet oder mit ihm interagiert. Bevorzugt ist das Nachweisagens fähig, an die Antigenbindungsregion des Autoantikörpers zu binden. Folglich ist in einer bevorzugten Ausführungsform der vorliegenden Erfindung das Nachweisagens das Volllängen-Autoantigen, gegen das der Autoantikörper gerichtet ist, oder ein Fragment davon. Zum Beispiel ist das Nachweisagens das Knorpelmatrixprotein (Autoantigen) selbst, gegen das der Autoantikörper gerichtet ist. So kann der Autoantikörper gegen TSP-4 durch Verwendung von Volllängen-TSP-4, gegen COMP durch Verwendung von Volllängen-COMP oder gegen CLEC3A durch Verwendung von Volllängen-CLEC3A detektiert werden. Das Nachweisagens kann auch ein Fragment des Volllängen-Autoantigens sein, sofern das Fragment von dem Autoantikörper gebunden wird (antigenes Fragment). Ein solches Fragment kann aus mindestens 5, mindestens 6, mindestens 7, mindestens 8, mindestens 9, mindestens 10, mindestens 15, mindestens 20, mindestens 25, mindestens 30, mindestens 35, mindestens 40, mindestens 45, mindestens 50 oder mehr als 50 Aminosäureresten des Volllängenautoantigens bestehen. Ein solches Fragment kann auch ein Degradationsprodukt sein, wie vorstehend definiert. Ein solches Degradationsprodukt kann ein Neoepitop umfassen, das durch die Degradation neu gebildet wird und bei dem nativen Protein nicht vorhanden ist. Zum Nachweis von anti-Kollagen II-Autoantikörpern umfasst die vorliegende Erfindung ein Nachweisagens, wie vorstehend definiert, das spezifisch gegen anti-Kollagen II-Autoantikörper gerichtet ist. Dieses kann ein Volllängen-Kollagen II-Protein oder ein Fragment davon, einschließlich eines Degradationsprodukts, sein. Alternativ ist ein weiteres Nachweisagens ein Protein oder Peptid mit einem Epitop, das eine Aminosäureidentität oder Aminosäurehomologie zu einem Epitop eines Volllängenautoantigens von mindestens 50%, 60%, 70%, 80%, 85%, 90%, 95% oder 100% aufweist, sofern dieses Epitop des Proteins oder Peptids spezifisch an einen Autoantikörper bindet, an den das Volllängenautoantigen (z. B. SEQ ID NR: 1, 2, 3 oder 4) bindet.

Das Autoantigen oder ein antigenes Fragment davon kann durch dem Fachmann bekannte Verfahren hergestellt werden. Beispielsweise können rekombinante DNA-Techniken verwendet werden, wobei ein DNA-Molekül, das das Autoantigen oder ein Fragment davon kodiert, gentechnisch in einen geeigneten Expressionsvektor eingeführt werden kann. Es kann auch vorteilhaft sein, Fusionsproteine zu konstruieren, die die Markierung, Immobilisierung oder Detektion des Antigens erleichtern (vgl. A Laboratory Manual (4. Auflage), Cold Spring Harbor Laboratory Press, 2012). Alternativ kann das Autoantigen oder ein antigenes Fragment davon aus natürlichen Quellen gereinigt werden, z.B. unter Verwendung von Proteintrennungstechniken, die im Stand der Technik gut bekannt sind. Solche Reinigungstechniken umfassen, sind aber nicht beschränkt auf, Molekularsiebchromatographie und/oder lonenaustauschchromatographie. Die Identifikation von antigenen Fragmenten des Autoantigens kann durch Verfahren erfolgen, die im Stand der Technik bekannt sind. Z.B. können Degradationsprodukte, die durch Autoantikörper erkannt werden, durch Immunblots identifiziert, gereinigt und sequenziert werden. Da Knorpelmatrixproteine im Stand der Technik bekannt sind, sind diese Proteine auch kommerziell erhältlich. Z.B. können TSP-4 und COMP von R & D Systems, CLEC3A DNA von Sino Biological und Kollagen II von Merck erworben werden.

Der Nachweis von einem Autoantikörper in einer Probe, die einem Subjekt entnommen wurde, kann durch eine Vielzahl von Arten erreicht werden, wie sie einem Fachmann bekannt sind. Beispielhafte Verfahren umfassen, sind jedoch nicht beschränkt auf, Antikörper-basierte (Immuntest-basierte) Testverfahren, einschließlich Western-Blot-Verfahren, Immunblot-Verfahren, Enzym-verknüpften Immunsorptionstest (ELISA), Sandwich-Immuntest, Radioimmuntest (RIA), Immunpräzipitations- und Dissoziations-verstärkten Lanthanidfluor-Immuntest (DELFIA), Präzipitin-Reaktion, Geldiffusions-Präzipitin-Reaktion, Immundiffusionstest, immunradiometrischen Test, Protein-A-Immuntest, Proteomics-Verfahren, Oberflächenplasmonresonanz (SPR), Chemilumineszenz, Fluoreszenzpolarisation, Phosphoreszenz, Immunhistochemie, Immunfluoreszenz, Mikrocytometrie, Mikroskopie, Fluoreszenz-aktivierte Zellsortierung (FACS), Durchflusszytometrie, Protein-Mikroarrays, Massenspektrometrie-basierten Techniken (einschließlich Flüssigkeitschromatographie, gekoppelt mit Tandem-Massenspektrometrie (LC-MS/MS), Nano-LC-MS/MS, Matrix-unterstützte Laser-Desorption/Ionisations-Massenspektrometrie (MALDI-MS), Matrix-unterstützte Laser-Desorption/Ionisation-Flugzeit (MALDI-TOF)-Massenspektrometrie, oberflächenverstärkte Laser-Desorptions/Ionisations-Massenspektrometrie (SELDI-MS), oberflächenverstärkte Laser-Desorptions/lonisations-Flugzeit (SELDI-TOF)-Massenspektrometrie, oberflächenverstärktes Affinitätseinfangen (SEAC), oberflächenverstärkte Reindesorption (SEND) oder oberflächenverstärkte photolabile Anheftungs- und Freisetzungs (SEPAR)-Massenspektrometrie.

Bevorzugt wird der Nachweis mit Hilfe eines Nachweisagens durchgeführt. Dabei wird eine Probe von einem Subjekt mit dem Antigen unter Bedingungen in Kontakt gebracht, die eine immunspezifische Antigen-Antikörper-Bindungsreaktion erlauben. Das Antigen kann in Lösung sein oder kann auf einem Träger immobilisiert sein. Umgekehrt kann der Autoantikörper auf einem Träger immobilisert sein. Falls in der Probe des Subjekts Autoantikörper vorhanden sind, erfolgt eine Bindung an das Antigen. Das bevorzugte Nachweisverfahren ist dabei ein Antikörper-basiertes Testverfahren, insbesondere ein Immunblot-Verfahren, bei dem z. B. das Matrixprotein in einem Gel aufgetrennt, auf eine Membran transferiert und mit einem Nachweisagens und markiertem anti-IgG-Antikörper detektiert wird. Weitere bevorzugte Nachweisverfahren sind Immunoassays" Slot/Dotblot-Verfahren, Lineblot-Verfahren, Fluoreszenznachweis auf Zellen oder Gewebe, Oberflächenplasmonresonanz-Verfahren oder Biochip/Proteinarray-Verfahren.

Unter "spezifisch bindet" oder "spezifisch interagiert" oder "immunspezifisch" wird hierin verstanden, dass das Nachweisagens im Wesentlichen nur an den Autoantikörper bindet, der nachgewiesen werden soll, oder mit ihm interagiert, während es an andere Substanzen nicht bindet oder nicht mit ihnen interagiert oder dies nur in geringem Ausmaß tut. "Im Wesentlichen" oder "geringes Ausmaß" bedeutet, dass das Nachweisagens an die andere Substanz zu weniger als 10, 5, 4, 3, 2 oder 1 % des Ausmaßes bindet, mit dem das Nachweisagens spezifisch an den Autoantikörper bindet.

Der Nachweis kann qualitativ und/oder quantitativ erfolgen. Dies kann die Hinzuziehung einer Referenz erfordern, um zu bestimmen, ob und/oder in welcher Konzentration der Autoantikörper in einer bestimmten Probenmenge vorliegt. Eine solche Referenz kann der Autoantikörper von bekannter Konzentration sein, der z. B. in einer Verdünnungsreihe vorliegt. Um die Konzentration des Autoantikörpers in einer Probe von einem Subjekt messen zu können, wird der Nachweis des Autoantikörpers in der Probe und der Verdünnungsreihe unter gleichen Bedingungen durchgeführt. Eine solche Referenz kann alternativ das Nachweisreagenz sein, das z. B. in einer Verdünnungsreihe vorliegt und mit Autoantikörpern definierter Konzentration/en bindet oder interagiert, um die Konzentration des Autoantikörpers von der Probe des Subjekts zu bestimmen.

Weiterhin betrifft die vorliegende Erfindung das Verfahren zur Diagnose von Arthrose oder des Risikos, Arthrose zu entwickeln, der vorliegenden Erfindung, wobei der Verlauf von Arthrose überwacht wird, umfassend den Nachweis des Autoantikörpers zu verschiedenen Zeitpunkten; oder wobei das Stadium von Arthrose diagnostiziert wird. Der Verlauf ist gekennzeichnet durch das unterschiedlich schnelle Voranschreiten der Erkrankung (chronisch progredient) z. B. in Abhängigkeit von der Reduktion der Risikofaktoren oder ggf. in Zukunft in Abhängigkeit von der Therapie mit den Krankheitsverlauf modifizierenden Medikamenten. Das Krankheitsstadium kann mittels Röntgen in Arthrose-Grade (Kellgren-Lawrence-Score) untergliedert werden. Typischerweise durchläuft der Arthrose-Patient im Verlauf seiner Erkrankung die Stadien nach Kellgren-Lawrence nacheinander.

Bei der Progression der Arthrose werden durch die Degradation der extrazellulären Knorpelmatrix Knorpel (Gelenkkapsel-, subchondrale Knochen-, Muskel- und Sehnen)-Proteine bzw. ihre Fragmente in die Synovia und den Blutkreislauf freigesetzt und somit dem Immunsystem präsentiert, welches abhängig von der Konzentration der Proteine oder Fragmente Autoantikörper bildet. Folglich deuten ansteigende Konzentrationen von Autoantikörpern auf eine Progression der Arthrose hin, während abnehmende Konzentrationen von Autoantikörpern eine verlangsamte oder gestoppte Progression anzeigen.

Die vorliegende Erfindung betrifft in einem 2. Aspekt weiterhin ein Verfahren zur Diagnose einer degenerativen Erkrankung des Skelettsystems oder des Risikos, eine degenerative Erkrankung des Skelettsystems zu entwickeln, bei einem Subjekt, umfassend das Nachweisen von einem Autoantikörper gegen Thrombospondin-4 (TSP-4) oder ein Degradationsprodukt oder ein Fragment davon oder das Nachweisen von einem Autoantikörper gegen COMP oder ein Degradationsprodukt oder ein Fragment davon in einer von dem Subjekt stammenden Probe.

In einer Ausführungsform davon betrifft die vorliegende Erfindung dieses Verfahren des 2. Aspekts, wobei die degenerative Erkrankung des Skelettsystems rheumatoide Arthritis oder Arthrose umfasst. In einer Ausführungsform davon betrifft die vorliegende Erfindung dieses Verfahren des 2. Aspekts, wobei dieses Verfahren das Nachweisen von mindestens zwei Autoantikörpern umfasst; bevorzugt wobei dieses Verfahren das Nachweisen von einem Autoantikörper gegen Thrombospondin-4 (TSP-4) oder ein Degradationsprodukt oder ein Fragment davon und das Nachweisen von einem Autoantikörper gegen COMP oder ein Degradationsprodukt oder ein Fragment davon umfasst; oder bevorzugt wobei dieses Verfahren das Nachweisen von einem Autoantikörper gegen Thrombospondin-4 (TSP-4) oder ein Degradationsprodukt oder ein Fragment davon und das Nachweisen von einem Autoantikörper gegen CLEC3A oder ein Degradationsprodukt oder ein Fragment davon umfasst; stärker bevorzugt wobei dieses Verfahren das Nachweisen von einem Autoantikörper gegen Thrombospondin-4 (TSP-4) oder ein Degradationsprodukt oder ein Fragment davon, das Nachweisen von einem Autoantikörper gegen COMP oder ein Degradationsprodukt oder ein Fragment davon und das Nachweisen von einem Autoantikörper gegen CLEC3A oder ein Degradationsprodukt oder ein Fragment davon umfasst.

In einer Ausführungsform davon betrifft die vorliegende Erfindung dieses Verfahren des 2. Aspekts, wobei der Autoantikörper gegen ein Neoepitop eines Degradationsprodukts eines Matrixproteins gerichtet ist.

In einer Ausführungsform davon betrifft die vorliegende Erfindung dieses Verfahren des 2. Aspekts, wobei das Nachweisen mit einem Nachweisagens erfolgt, bevorzugt wobei das Nachweisagens fähig ist, an die Antigenbindungsregion des Autoantikörpers zu binden, stärker bevorzugt wobei das Nachweisagens TSP-4 oder ein Degradationsprodukt oder ein Fragment davon, COMP oder ein Degradationsprodukt oder ein Fragment davon und/oder CLEC3A oder ein Degradationsprodukt oder ein Fragment davon ist.

In einer Ausführungsform davon betrifft die vorliegende Erfindung dieses Verfahren des 2. Aspekts, wobei die Probe Körperflüssigkeit, bevorzugt Blut, Serum, Blutplasma, Synovialflüssigkeit oder Urin, Muskel- oder Knorpelgewebe, Synovialmembran oder Sehne ist.

Unter "degenerativer Erkrankung" versteht man allgemein die mit einer fortschreitenden Degeneration einhergehende Erkrankung. Degeneration bezeichnet funktionelle und/oder morphologische Veränderungen einer Zelle, eines Gewebes, eines Organs oder des gesamten Organismus, die im Vergleich zur vollen physiologischen Leistungsfähigkeit eine Verschlechterung zum Beispiel durch Rückbildung, Verfall, Abbau oder Funktionsverlust anlagebedingt oder aufgrund von chronischen Schädigungsfaktoren darstellen. Unter "degenerativer Erkrankung des Skelettsystems", wie hierin verwendet, versteht man die Degeneration der Wirbelsäule und der Gelenke bzw. ihrer Knorpelbestandteile, bevorzugt RA oder Arthrose.

Die Erfindung betrifft in einem 3. Aspekt weiterhin ein Verfahren zur Diagnose von Arthrose oder des Risikos, Arthrose zu entwickeln, bei einem Subjekt, umfassend das Ausschließen des Vorliegens eines Autoantikörpers gegen Kollagen II oder ein Degradationsprodukt oder ein Fragment davon, bevorzugt wobei das Verfahren das Nachweisen, d.h. den Schritt des Nachweisens, eines Autoantikörpers gegen Kollagen II oder ein Degradationsprodukt oder ein Fragment davon umfasst, stärker bevorzugt wobei das Verfahren das Nachweisen eines Autoantikörpers gegen Kollagen II oder ein Degradationsprodukt oder ein Fragment davon mit einem Nachweisagens zum Nachweisen eines Autoantikörpers gegen Kollagen II oder ein Degradationsprodukt oder ein Fragment davon umfasst.

In einer Ausführungsform davon betrifft die vorliegende Erfindung dieses Verfahren des 3. Aspekts, wobei die Arthrose zusätzlich durch herkömmliche Methoden diagnostiziert wird.

In einer Ausführungsform davon betrifft die Erfindung das vorstehende Verfahren des 3. Aspekts zur Diagnose von Arthrose oder des Risikos, Arthrose zu entwickeln, bei einem Subjekt, umfassend das Ausschließen des Vorliegens eines Autoantikörpers gegen Kollagen II oder ein Degradationsprodukt oder ein Fragment davon, wobei bei fehlendem Nachweis eines Autoantikörpers gegen Kollagen II oder ein Degradationsprodukt oder ein Fragment davon das Subjekt diagnostiziert wird, dass es Arthrose aufweist, während bei Nachweis eines Autoantikörpers gegen Kollagen II oder ein Degradationsprodukt oder ein Fragment davon das Subjekt diagnostiziert wird, dass es keine Arthrose aufweist.

Das vorstehende Verfahren, umfassend den Nachweis eines Autoantikörpers gegen Kollagen II oder ein Degradationsprodukt oder ein Fragment davon, kann gemäß der vorliegenden Erfindung alleine verwendet werden, um das Vorliegen von Arthrose oder das Nicht-Vorliegen von Arthrose bei einem Subjekt nachzuweisen. Alternativ kann dieses Verfahren zusätzlich zu herkömmlichen Diagnoseverfahren von Arthrose oder zusätzlich zu dem Verfahren zur Diagnose von Arthrose oder des Risikos, Arthrose zu entwickeln, der vorliegenden Erfindung unter Nachweis eines Autoantikörpers, der mit Arthrose assoziiert ist, verwendet werden. Dabei kann der Nicht-Nachweis oder der Nachweis eines Autoantikörpers gegen Kollagen II oder ein Degradationsprodukt oder ein Fragment davon als zusätzliches Indiz gewertet werden, dass Arthrose (Nicht-Nachweis) bzw. dass keine Arthrose (Nachweis) bei dem Subjekt vorliegt.

In einer Ausführungsform davon betrifft die vorliegende Erfindung dieses Verfahren des 3. Aspekts, wobei die Probe Körperflüssigkeit, bevorzugt Blut, Serum, Blutplasma, Synovialflüssigkeit oder Urin, Muskel- oder Knorpelgewebe, Synovialmembran oder Sehne ist.

Die vorliegende Erfindung betrifft weiterhin einen Kit zur Diagnose von Arthrose oder des Risikos, Arthrose zu entwickeln, bei einem Subjekt, wobei das Subjekt keine rheumatoide Arthritis aufweist, umfassend ein Nachweisagens zum Nachweisen von einem Autoantikörper, der mit Arthrose assoziiert ist, bevorzugt wobei das Nachweisagens ein Nachweisagens zum Nachweisen von einem Autoantikörper gegen TSP-4 oder ein Degradationsprodukt oder ein Fragment davon oder ein Nachweisagens zum Nachweisen von einem Autoantikörper gegen COMP oder ein Degradationsprodukt oder ein Fragment davon umfasst.

Die vorliegende Erfindung betrifft weiterhin einen Kit zur Diagnose einer degenerativen Erkrankung des Skelettsystems oder des Risikos, eine degenerative Erkrankung des Skelettsystems zu entwickeln, bei einem Subjekt, umfassend ein Nachweisagens zum Nachweisen von einem Autoantikörper gegen TSP-4 oder ein Degradationsprodukt oder ein Fragment davon oder ein Nachweisagens zum Nachweisen von einem Autoantikörper gegen COMP oder ein Degradationsprodukt oder ein Fragment davon umfasst, bevorzugt wobei die degenerative Erkrankung des Skelettsystems rheumatoide Arthritis oder Arthrose (inklusive Osteochondrose) umfasst.

In einer Ausführungsform davon betrifft die vorliegende Erfindung den Kit, wie vorstehend beschrieben, wobei das Nachweisagens mindestens zwei Nachweisagentien zum Nachweisen von mindestens zwei Autoantikörpern umfasst, bevorzugt wobei das Nachweisagens ein Nachweisagens zum Nachweisen von einem Autoantikörper gegen TSP-4 oder ein Degradationsprodukt oder ein Fragment davon und ein Nachweisagens das Nachweisen von einem Autoantikörper gegen COMP oder ein Degradationsprodukt oder ein Fragment davon umfasst; oder bevorzugt wobei das Nachweisagens ein Nachweisagens zum Nachweisen von einem Autoantikörper gegen TSP-4 oder ein Degradationsprodukt oder ein Fragment davon und ein Nachweisagens das Nachweisen von einem Autoantikörper gegen CLEC3A oder ein Degradationsprodukt oder ein Fragment davon umfasst; stärker bevorzugt wobei das Nachweisagens ein Nachweisagens zum Nachweisen von einem Autoantikörper gegen TSP-4 oder ein Degradationsprodukt oder ein Fragment davon, ein Nachweisagens zum Nachweisen von einem Autoantikörper gegen COMP oder ein Degradationsprodukt oder ein Fragment davon und ein Nachweisagens zum Nachweisen von einem Autoantikörper gegen CLEC3A oder ein Degradationsprodukt oder ein Fragment davon umfasst.

In einer Ausführungsform davon betrifft die vorliegende Erfindung den Kit, wobei der Kit weiterhin ein Nachweisagens zum Nachweisen von einem Autoantikörper gegen Kollagen II oder ein Degradationsprodukt oder ein Fragment davon umfasst.

Die vorliegende Erfindung betrifft weiterhin die Verwendung des Kits zur Diagnose von Arthrose oder des Risikos, Arthrose zu entwickeln, bei einem Subjekt, wobei das Subjekt keine rheumatoide Arthritis aufweist; oder zur Diagnose von einer degenerativen Erkrankung des Skelettsystems oder des Risikos, eine degenerative Erkrankung des Skelettsystems zu entwickeln, bei einem Subjekt, bevorzugt wobei die degenerative Erkrankung des Skelettsystems rheumatoide Arthritis oder Arthrose (inklusive Osteochondrose) umfasst.

Der Kit kann weiterhin zu dem Nachweisagens einen festen Träger wie eine Membran (z.B. PVDF-Membran), einen Chip, einen Sensor, eine Mikrotiterplatte, Kügelchen, Harz, Glas, Keramik oder Metall, das mit einem synthetischen Polymer, Glas, Keramik, synthetischen Polymeren und Biopolymeren beschichtet ist, z.B. quervernetztem Dextran oder Agarose, Nylon, Polyethylen oder Polystyrol umfassen. Das Nachweisagens kann bereits auf dem festen Träger immobilisiert sein, oder Träger und Nachweisagens sind in getrennten Behältern enthalten und das Nachweisagens wird vor der Verwendung auf den festen Träger aufgetragen und immobilisiert. Das Nachweisagens liegt bevorzugt in einer oder mehreren definierten Mengen vor, um die Abschätzung der Menge des Autoantikörpers in der Subjektprobe zu erlauben. Der Kit kann weiterhin Bestandteile umfassen, die zum Ausführen des Verfahrens der vorliegenden Erfindung geeignet sind, beispielsweise Waschlösungen oder Lösungen oder Vorrichtungen, um ein bestimmtes Nachweisverfahren auszuführen, z.B. Massenspektrometriesonden für SELDI wie ProteinChip®-Arrays oder auf Faser basierende Sensorvorrichtungen, etc. Eine Waschlösung kann zum Abwaschen von Probenresten nach deren Auftragen auf den festen Träger, an den das Nachweisagens immobilisiert wird, oder zum Abwaschen von Resten des Nachweisagens, das zur Immobilisierung auf einen festen Träger aufgebracht wird, verwendet werden. Der Kit kann mehr als eine Art von Nachweisagens gegen verschiedene Autoantikörper umfassen. Z. B. kann der Kit mehrere Knorpelmatrixproteine, die mit Arthrose assoziiert sind, oder Degradationsprodukte oder Fragmente davon, z.B. Kombinationen von TSP-4 und COMP, von TSP-4 und CLEC3A oder von TSP-4 und COMP und CLEC3A, gegebenenfalls mit Kollagen II, oder Degradationsprodukte oder Fragmente davon, in getrennten oder gleichen Behältern umfassen.

In einer weiteren Ausführungsform kann der Kit Anweisungen für die Ausführung des Verfahrens der vorliegenden Erfindung und/oder die Interpretation der Ergebnisse umfassen, z.B. in Form eines Beipackzettels oder einer Broschüre. Zum Beispiel können die Anweisungen den Verbraucher informieren, wie die Probe zu gewinnen, ggf. das Nachweisagens auf dem festen Träger zu immobilisieren, der Träger zu waschen und/oder die Probe aufzutragen ist. Der Kit kann zusätzlich Referenz-Nachweisagens einer definierten Menge oder in mehreren definierten Mengen enthalten, wobei das Referenz-Nachweisagens die Abschätzung der Konzentration des Autoantikörpers in der Probe erlaubt. Das Referenz-Nachweisagens ist dabei dasselbe wie das eigentliche Nachweisagens, z.B. TSP-4, TSP-4 und COMP, TSP-4 und CLEC3A oder TSP-4 und COMP und CLEC3A, gegebenenfalls mit Kollagen II, oder Degradationsprodukte oder Fragmente davon. Wenn die Referenz ein Referenz-Nachweisagens ist, kann der Kit zusätzlich Autoantikörper einer oder mehrerer bestimmten Konzentrationen enthalten, die ggf. bereits auf einem festen Träger immobilisiert sein können, um den Vergleich mit dem Autoantikörper von der Probe des Subjekts zu erlauben. Oder der Kit kann zusätzlich Referenz-Autoantikörper einer bestimmten Menge oder in mehreren bestimmten Mengen enthalten, ggf. in einem zusätzlichen Behältnis oder bereits auf einem festen Träger immobilisiert, wobei der Autoantikörper die Abschätzung der Konzentration des Autoantikörpers in der Probe erlaubt. Der/die Referenz-Autoantikörper ist/sind dabei derselbe/dieselben wie der/die eigentliche/n Autoantikörper, z.B. gegen TSP-4, TSP-4 und COMP, TSP-4 und CLEC3A oder TSP-4 und COMP und CLEC3A, und gegebenenfalls gegen Kollagen II, oder Degradationsprodukte oder Fragmente davon.

Die vorliegende Erfindung betrifft weiterhin einen Wirkstoff zur Verwendung zur Behandlung oder Vorbeugung einer autoimmun-assoziierten Arthrose bei einem Subjekt, bevorzugt wobei der Wirkstoff Rituximab ist. Das Subjekt ist dabei das durch das vorliegende Verfahren diagnostizierte Subjekt.

Aktuell existiert keine medikamentöse Therapie, welche die Progression der Arthrose beeinflusst. Allerdings kommen nicht steroidale Antirheumatika (z. B. Ibuprofen, Diclofenac, Indometacin und Napropxen und Coxibe) zur Linderung der Beschwerden zum Einsatz, ggf. bei starken Schmerzen auch Opioide. In Phasen der akuten Gelenkentzündung kommt ggf. eine intraartikuläre Gabe von z. B. Triamcinolonhexacetonid (Glukocorticoid) in Frage. Die vorliegende Erfindung ist auf den Nachweis von Autoantikörpern, die mit Arthrose assoziiert sind, gerichtet. Im Tierversuch wurde nachgewiesen, dass Autoantikörper gegen Knorpelmatrixproteine wie COMP eine pathophysiologische Bedeutung haben und zu schwerer chronischer Arthritis führen können. Somit ist das Vorliegen von Autoantikörpern, die mit Arthrose assoziiert sind, bei einem Subjekt ein Hinweis auf eine pathophysiologische Rolle dieser Autoantikörper beim Entstehen, beim Aufweisen und bei der Progression von Arthrose. Die Pathophysiologie der Autoantikörper erlaubt deshalb die Anwendung einer spezifischen, individuellen Therapie (personalisierte Therapie), die eine allgemeine Immuntherapie zur Unterdrückung der Bildung von Autoantikörpern als auch eine Therapie zur Unterdrückung der Bildung von spezifischen Autoantikörpern, die mit Arthrose assoziiert sind, einschließt. Vorstellbar wäre eine "targeted-Therapie", bei welcher ein Zytostatikum durch spezifische Bindung eines Antigens zur Immunzelle transportiert wird und diese dabei abgetötet wird. Folglich ist bei Patienten, bei denen Autoantikörper, die mit Arthrose assoziiert sind, nachgewiesen werden, jede Art von Therapie wirksam, die die Konzentration der Autoantikörper reduziert, z. B. indem sie die Bildung neuer Autoantikörper hemmt. Deshalb ist eine Form von Therapie im Rahmen der vorliegenden Erfindung die Immuntherapie bzw. die Autoimmuntherapie, da sie gegen Autoantikörper gerichtet ist. Immuntherapien sind Behandlungsformen, bei denen das Immunsystem beeinflusst wird. Hierbei kommen in Abhängigkeit von der Erkrankung modulierende (stimulierende und supprimierende) oder substituierende (ersetzende) Verfahren zur Anwendung. Bei Autoimmunkrankheiten kommen im Allgemeinen supprimierende Verfahren, die immunologische Prozesse unterdrücken, z. B. die Gabe von Immunsuppressiva, zur Anwendung, um unerwünschte Reaktionen des Immunsystems zu hemmen. Gängige Immunsuppressiva sind z. B. Ciclosporin A, Tacrolimus, Sirolimus, Azathioprin und Methotrexat. Immunsuppressive Verfahren finden auch im Rahmen der vorliegenden Erfindung bei der Behandlung von immun-assoziierter Arthrose Anwendung. Bevorzugt ist eine im Rahmen der vorliegenden Erfindung verwendete Therapie auf eine Reduktion der Plasma- oder B-Zellen ausgerichtet, wobei die Therapie bevorzugt durch eine intraartikuläre oder systemische, besonders bevorzugt intraartikuläre, Gabe eines Wirkstoffs erfolgt, der eine solche Reduktion bewirken kann, wie z. B. Rituximab, Ofatumumab, Ocrelizumab und Epratuzumab bevorzugt Rituximab. Eine andere Form der Therapie ist eine Behandlung mit Wirkstoffen wie Antikörper, die B-Zell-stimulierende Zytokine neutraliseren oder hemmen, z. B. Anakinra, Infliximab, Adalimumab Etanercept, Tocilizumab. Eine weitere Form der Therapie ist die Inhibierung der Aktivierung und Proliferation von B-Zellen durch Inhibierung der Signaltransduktion durch z. B. Belimumab und Atacicept. Eine wichtige Rolle bei der Aktivierung und Proliferation von B-Zellen kommt dabei der Wnt-Signaltransduktion zu. Ein wirksamer Inhibitor der Wnt-Signaltransduktionskaskade ist SM04690, von dem in präklinischen Studien gezeigt wurde, dass er eine Wirkung als "disease-modifying drug" (DMOAD) aufweist. Ein weiteres, bereits zugelassenes Medikament ist Fluoxetin (zugelassen zur Behandlung von Depressionen), welches die Wnt-Siganltransduktionskaskade und damit auch die Proliferation von B-Zellen inhibiert. Folglich ist ein "Wirkstoff zur Verwendung zur Behandlung oder Vorbeugung einer autoimmun-assoziierten Arthrose" ein Immunsuppressivum, ein Wirkstoff zur Reduktion von Plasma- oder B-Zellen, z. B. Rituximab, ein Wirkstoff wie ein Antikörper, der B-Zell-stimulierende Zytokine neutralisert oder hemmt, und/oder ein Wirkstoff, der die Signaltransduktion hemmt, die zur Aktivierung und Proliferation von B-Zellen führt, z. B. ein Wirkstoff, der die Wnt-Signaltransduktion hemmt, z. B. SM04690 oder Fluoxetin.

Bei einer aktivierten Arthrose kommt es nicht selten zur intraartikulären Applikation von Cortisonpräparaten. Cortisonpräparate sorgen für eine Stimulation der T-Helfer-Zellen, u.a. der TH2-Zellen, welche wiederum zur Aktivierung und Proliferation von B-Zellen führen. Daher wäre bei Patienten mit Autoantikörpern, die mit Arthrose assoziiert sind, oder mit mehreren Autoantikörpern, die mit Arthrose assoziiert sind, die Gabe von Cortisonpräparaten eher nachteilig. Subjekte, die von einer Wirkstoffbehandlung profitieren, sind solche, bei denen mit den Diagnoseverfahren der vorliegenden Erfindung Autoantikörper, die mit Arthrose assoziiert sind, nachgewiesen werden. Die vorliegende Erfindung betrifft weiterhin ein Verfahren zur Diagnose von Arthrose oder des Risikos, Arthrose zu entwickeln, oder zur Diagnose einer degenerativen Erkrankung des Skelettsystems oder des Risikos, eine degenerative Erkrankung des Skelettsystems zu entwickeln, gemäß der vorliegenden Erfindung, wobei das Verfahren zur Therapieauswahl eingesetzt wird. Wenn in dem Verfahren der Diagnose von Arthrose oder des Risikos, Arthrose zu entwickeln, oder der Diagnose einer degenerativen Erkrankung des Skelettsystems oder des Risikos, eine degenerative Erkrankung des Skelettsystems zu entwickeln, ein Autoantikörper nachgewiesen wird, der mit Arthrose bzw. der degenerativen Erkrankung des Skelettsystems assoziiert ist, können die betroffenen Subjekte für eine Therapie ausgewählt werden, bei der die Arthrose oder die degenerative Erkrankung des Skelettsystems durch Reduktion der Konzentration der Autoantikörper behandelt oder ihr vorgebeugt wird. Somit bezieht sich der Begriff "Therapieauswahl" auf die Auswahl von Subjekten, bei denen eine Arthrose oder degenerative Erkrankung des Skelettsystems gemäß der Erfindung diagnostiziert wurde, für eine Therapie, bei der die Konzentration der Autoantikörper reduziert wird, wie vorstehend beschrieben. Insbesondere beinhaltet die Therapie einen Wirkstoff zur Verwendung zur Behandlung oder Vorbeugung einer autoimmun-assoziierten Arthrose bei einem Subjekt, bevorzugt wobei der Wirkstoff ein Antikörper gegen B-Zellen (z.B. Rituximab) oder ein Wnt-Signalisierungs-Inhibitor (z.B. Fluoxetin oder SM04690) ist. Die Behandlung erfolgt bevorzugt intraartikulär.

Weitere Aspekte der Erfindung sind:
Die vorliegende Erfindung ist auf ein Verfahren zum Nachweis eines Autoantikörpers, der mit Arthrose assoziiert ist, oder zur Diagnose von Arthrose oder des Risikos, Arthrose zu entwickeln, bei einem Subjekt, wobei das Subjekt keine rheumatoide Arthritis aufweist, gerichtet, wobei das Verfahren umfasst:
   a) Gewinnen einer Probe von dem Subjekt; und
   b) Nachweisen, ob ein Autoantikörper, der mit Arthrose assoziiert ist, in der Probe vorhanden ist, wobei der Nachweis das Inkontaktbringen der Probe mit einem Nachweisagens und Nachweisen der Bindung zwischen dem Autoantikörper und dem Nachweisagens umfassen kann.

Das vorstehende Verfahren kann weiterhin die Schritte umfassen:
i) Gewinnen einer Probe von dem Subjekt;
ii) Nachweisen, ob ein Autoantikörper gegen Kollagen II oder ein Degradationsprodukt oder Fragment davon in der Probe vorhanden ist, wobei der Nachweis das Inkontaktbringen der Probe mit einem Nachweisagens und Nachweisen der Bindung zwischen dem Autoantikörper und dem Nachweisagens umfassen kann.

Die vorliegende Erfindung ist auch auf ein Verfahren zur Diagnose von Arthrose oder des Risikos, Arthrose zu entwickeln, bei einem Subjekt, wobei das Subjekt keine rheumatoide Arthritis aufweist, gerichtet, wobei das Verfahren umfassen kann;
a) Gewinnen einer Probe von dem Subjekt;
b) Nachweisen, ob ein Autoantikörper, der mit Arthrose assoziiert ist, in der Probe vorhanden ist, wobei der Nachweis durch Inkontaktbringen der Probe mit einem Nachweisagens und Nachweisen der Bindung zwischen dem Autoantikörper und dem Nachweisagens umfasst; und
c) Diagnostizieren des Subjekts, dass es Arthrose oder ein Risiko, Arthrose zu entwickeln, aufweist, wenn ein Autoantikörper, der mit Arthrose assoziiert ist, in der Probe nachgewiesen wird.

Das vorstehende Verfahren kann weiterhin die Schritte umfassen:
i) Gewinnen einer Probe von dem Subjekt;
ii) Nachweisen, ob ein Autoantikörper gegen Kollagen II oder ein Degradationsprodukt oder Fragment davon in der Probe vorhanden ist, wobei der Nachweis das Inkontaktbringen der Probe mit einem Nachweisagens und Nachweisen der Bindung zwischen dem Autoantikörper und dem Nachweisagens umfassen kann;
iii) Diagnostizieren des Subjekts, dass es Arthrose oder ein Risiko, Arthrose zu entwickeln, aufweist, wenn ein Autoantikörper gegen Kollagen II oder ein Degradationsprodukt oder Fragment davon in der Probe nicht nachgewiesen wird.

Die vorliegende Erfindung ist auch auf ein Verfahren zur Diagnose von Arthrose oder des Risikos, Arthrose zu entwickeln, und Behandlung von Arthrose bei einem Subjekt, wobei das Subjekt keine rheumatoide Arthritis aufweist, gerichtet, wobei das Verfahren umfasst:
a) Gewinnen einer Probe von einem Subjekt;
b) Nachweisen, ob ein Autoantikörper, der mit Arthrose assoziiert ist, in der Probe vorhanden ist;
c) Diagnostizieren des Subjekts, dass es Arthrose oder ein Risiko, Arthrose zu entwickeln, aufweist, wenn ein Autoantikörper, der mit Arthrose assoziiert ist, in der Probe nachgewiesen wird; und
d) Verabreichen einer Behandlung von Arthrose an das diagnostizierte Subjekt.

Das vorstehende Verfahren kann weiterhin die Schritte umfassen:
i) Gewinnen einer Probe von dem Subjekt;
ii) Nachweisen, ob ein Autoantikörper gegen Kollagen II oder ein Degradationsprodukt oder Fragment davon in der Probe vorhanden ist;
iii) Diagnostizieren des Subjekts, dass es Arthrose oder ein Risiko, Arthrose zu entwickeln, aufweist, wenn ein Autoantikörper gegen Kollagen II oder ein Degradationsprodukt oder Fragment davon in der Probe nicht nachgewiesen wird.

Die vorliegende Erfindung ist auch auf ein Verfahren zur Behandlung oder Vorbeugung von Arthrose bei einem Subjekt, wobei das Subjekt keine rheumatoide Arthritis aufweist, gerichtet, wobei das Verfahren das Verabreichen einer Behandlung von Arthrose an ein im Rahmen der vorliegenden Erfindung diagnostiziertes Subjekt umfasst.

Die vorliegende Erfindung ist auch auf die Verwendung eines Wirkstoffs zur Herstellung eines Arzneimittels zur Behandlung einer autoimmun-assoziierten Arthrose bei einem Subjekt gerichtet.

Die Behandlung kann dabei eine Behandlung umfassen, bei der die Konzentration der Autoantikörper, die mit Arthrose assoziiert sind, reduziert wird. Zum Beispiel umfasst die Behandlung ein Immunsuppressivum (z. B. Ciclosporin A, Tacrolimus, Sirolimus, Azathioprin und Methotrexat), einen Wirkstoff zur Reduktion von Plasma- oder B-Zellen, z. B. Rituximab, Ofatumumab, Ocrelizumab und Epratuzumab, einen Wirkstoff wie einen Antikörper, der B-Zell-stimulierende Zytokine neutralisert oder hemmt, z. B. Anakinra, Infliximab, Adalimumab Etanercept, Tocilizumab, und/oder einen Wirkstoff, der die Signaltransduktion hemmt, die zur Aktivierung und Proliferation von B-Zellen führt, z. B. einen Wirkstoff, der die Signaltransduktion hemmt, (z.B. Belimumab und Atacicept) wie z. B. einen Wirkstoff, der die Wnt-Signaltransduktion hemmt, z. B. SM04690 oder Fluoxetin.

Die vorliegende Erfindung ist auf ein Verfahren zum Nachweis eines Autoantikörpers, der mit einer degenerativen Erkrankung des Skelettsystems assoziiert ist, oder zur Diagnose von einer degenerativen Erkrankung des Skelettsystems oder des Risikos, eine degenerative Erkrankung des Skelettsystems zu entwickeln, bei einem Subjekt gerichtet, wobei das Verfahren umfasst:
a) Gewinnen einer Probe von dem Subjekt; und
b) Nachweisen, ob ein Autoantikörper, der mit einer degenerativen Erkrankung des Skelettsystems assoziiert ist, in der Probe vorhanden ist, wobei der Nachweis das Inkontaktbringen der Probe mit einem Nachweisagens und Nachweisen der Bindung zwischen dem Autoantikörper und dem Nachweisagens umfassen kann.

Die vorliegende Erfindung ist auch auf ein Verfahren zur Diagnose von einer degenerativen Erkrankung des Skelettsystems oder des Risikos, eine degenerative Erkrankung des Skelettsystems zu entwickeln, bei einem Subjekt gerichtet, wobei das Verfahren umfasst:
a) Gewinnen einer Probe von dem Subjekt;
b) Nachweisen, ob ein Autoantikörper, der mit einer degenerativen Erkrankung des Skelettsystems assoziiert ist, in der Probe vorhanden ist, wobei der Nachweis das Inkontaktbringen der Probe mit einem Nachweisagens und Nachweisen der Bindung zwischen dem Autoantikörper und dem Nachweisagens umfassen kann; und
c) Diagnostizieren des Subjekts, dass es eine degenerative Erkrankung des Skelettsystems oder ein Risiko, eine degenerative Erkrankung des Skelettsystems zu entwickeln, aufweist, wenn ein Autoantikörper, der mit einer degenerativen Erkrankung des Skelettsystems assoziiert ist, in der Probe nachgewiesen wird.

Die vorliegende Erfindung ist auch auf ein Verfahren zur Diagnose von einer degenerativen Erkrankung des Skelettsystems oder des Risikos, eine degenerative Erkrankung des Skelettsystems zu entwickeln, und Behandlung von einer degenerativen Erkrankung des Skelettsystems bei einem Subjekt gerichtet, wobei das Verfahren umfasst:
a) Gewinnen einer Probe von einem Subjekt;
b) Nachweisen, ob ein Autoantikörper, der mit einer degenerativen Erkrankung des Skelettsystems assoziiert ist, in der Probe vorhanden ist, wobei der Nachweis das Inkontaktbringen der Probe mit einem Nachweisagens und Nachweisen der Bindung zwischen dem Autoantikörper und dem Nachweisagens umfassen kann;
c) Diagnostizieren des Subjekts, dass es eine degenerative Erkrankung des Skelettsystems oder ein Risiko, eine degenerative Erkrankung des Skelettsystems zu entwickeln, aufweist, wenn ein Autoantikörper, der mit einer degenerativen Erkrankung des Skelettsystems assoziiert ist, in der Probe nachgewiesen wird; und
d) Verabreichen einer Behandlung der degenerativen Erkrankung des Skelettsystems an das diagnostizierte Subjekt.

Die vorliegende Erfindung ist auch auf ein Verfahren zur Behandlung von oder Vorbeugung einer degenerativen Erkrankung des Skelettsystems bei einem Subjekt gerichtet, wobei das Verfahren das Verabreichen einer Behandlung der degenerativen Erkrankung des Skelettsystems an ein im Rahmen der vorliegenden Erfindung diagnostiziertes Subjekt umfasst.

Die vorliegende Erfindung ist auch auf die Verwendung eines Wirkstoffs zur Herstellung eines Arzneimittels zur Behandlung einer degenerativen Erkrankung des Skelettsystems bei einem Subjekt gerichtet.

Ein Arzneimittel umfasst den Wirkstoff zur Behandlung einer Krankheit und einen pharmazeutisch verträglichen Träger, der im Fachgebiet bekannt ist. Das Arzneimittel kann für verschiedene Verabreichungsformen formuliert sein, z. B. für eine lokale intraartikuläre oder systemische Gabe (oral, intravenös, subkutan, intramuskulär). Der Wirkstoff wird in einer wirksamen Menge verabreicht, die vom Fachmann bestimmt werden kann oder dem Fachmann bekannt ist, wobei sich die Menge des Wirkstoffs an bereits bekannten Mengen dieses Wirkstoffs zur Behandlung anderer Krankheiten orientieren kann. Dabei hängt die wirksame Menge von verschiedenen Faktoren wie Dosierungsform, Alter, Körpergewicht, Geschlecht, Dauer der Behandlung und ähnlichen Faktoren ab. Das Arzneimittel kann als Lösung, Suspension, Tablette, Kapseln oder Pulver, desweiteren noch als Paste, Salbe, Öl, Creme, Lotion, Schaum, Gel oder Zäpfchen, vorliegen.

Die Behandlung kann dabei eine Behandlung umfassen, bei der die Konzentration der Autoantikörper, die mit der degenerativen Erkrankung des Skelettsystems assoziiert sind, reduziert wird. Zum Beispiel umfasst die Behandlung ein Immunsuppressivum, einen Wirkstoff zur Reduktion von Plasma- oder B-Zellen, z. B. Rituximab, einen Wirkstoff wie einen Antikörper, der B-Zell-stimulierende Zytokine neutralisert oder hemmt, und/oder einen Wirkstoff, der die Signaltransduktion hemmt, die zur Aktivierung und Proliferation von B-Zellen führt, z. B. einen Wirkstoff, der die Wnt-Signaltransduktion hemmt, z. B. SM04690 oder Fluoxetin. Diese Art der Behandlung ist insbesondere für die Behandlung von Arthrose wirksam. Auch im Falle von RA erfolgt gemäß der Erfindung bei Nachweis eines Autoantikörpers gegen TSP-4 oder ein Degradationsprodukt oder Fragment davon oder gegen COMP oder ein Degradationsprodukt oder Fragment davon die Behandlung, wie vorstehend aufgezeigt, also mit einem Immunsuppressivum, einem Wirkstoff zur Reduktion von Plasma- oder B-Zellen, z. B. Rituximab, einem Wirkstoff wie einen Antikörper, der B-Zell-stimulierende Zytokine neutralisert oder hemmt, und/oder einem Wirkstoff, der die Signaltransduktion hemmt, die zur Aktivierung und Proliferation von B-Zellen führt, z. B. einen Wirkstoff, der die Wnt-Signaltransduktion hemmt, z. B. SM04690 oder Fluoxetin. Dabei ist eine solche Behandlung, bevorzugt mit Rituximab, gegenüber der gegenwärtigen Behandlung mit, in dieser Reihenfolge, (1) Methotrexat (MTX) als Monotherapie oder (2) in Kombination mit oder alternativ zu der Gabe von Biologicals (völlig oder nahezu mit körpereigenen Substanzen identische Präparate) und (3) Gabe von Rituximab bevorzugt, um Gelenkschäden in der Initialphase zu vermeiden, zu bevorzugen.

Die vorliegende Erfindung ist auf ein Verfahren zum Nachweis eines Autoantikörpers gegen Kollagen II oder ein Degradationsprodukt oder Fragment davon oder zur Diagnose von Arthrose oder des Risikos, Arthrose zu entwickeln, bei einem Subjekt, wobei das Subjekt keine rheumatoide Arthritis aufweist, gerichtet, wobei das Verfahren umfasst:
i) Gewinnen einer Probe von dem Subjekt;
ii) Nachweisen, ob ein Autoantikörper gegen Kollagen II oder ein Degradationsprodukt oder Fragment davon in der Probe vorhanden ist, wobei der Nachweis das Inkontaktbringen der Probe mit einem Nachweisagens und Nachweisen der Bindung zwischen dem Autoantikörper und dem Nachweisagens umfassen kann.

Die vorliegende Erfindung ist auch auf ein Verfahren zur Diagnose von Arthrose oder des Risikos, Arthrose zu entwickeln, bei einem Subjekt, wobei das Subjekt keine rheumatoide Arthritis aufweist, gerichtet, wobei das Verfahren umfasst:
i) Gewinnen einer Probe von dem Subjekt;
ii) Nachweisen, ob ein Autoantikörper gegen Kollagen II oder ein Degradationsprodukt oder Fragment davon in der Probe vorhanden ist, wobei der Nachweis das Inkontaktbringen der Probe mit einem Nachweisagens und Nachweisen der Bindung zwischen dem Autoantikörper und dem Nachweisagens umfassen kann;
iii) Diagnostizieren des Subjekts, dass es Arthrose oder ein Risiko, Arthrose zu entwickeln, aufweist, wenn ein Autoantikörper gegen Kollagen II oder ein Degradationsprodukt oder Fragment davon in der Probe nicht nachgewiesen wird.

Die vorliegende Erfindung ist auch auf ein Verfahren zur Diagnose von Arthrose oder des Risikos, Arthrose zu entwickeln, und Behandlung von Arthrose bei einem Subjekt, wobei das Subjekt keine rheumatoide Arthritis aufweist, gerichtet, wobei das Verfahren umfasst:
i) Gewinnen einer Probe von dem Subjekt;
ii) Nachweisen, ob ein Autoantikörper gegen Kollagen II oder ein Degradationsprodukt oder Fragment davon in der Probe vorhanden ist, wobei der Nachweis das Inkontaktbringen der Probe mit einem Nachweisagens und Nachweisen der Bindung zwischen dem Autoantikörper und dem Nachweisagens umfassen kann;
iii) Diagnostizieren des Subjekts, dass es Arthrose oder ein Risiko, Arthrose zu entwickeln, aufweist, wenn ein Autoantikörper gegen Kollagen II oder ein Degradationsprodukt oder Fragment davon in der Probe nicht nachgewiesen wird;
iv) Verabreichen einer Behandlung von Arthrose an das Subjekt.

Die Behandlung ist, wie vorstehend beschrieben, und/oder eine herkömmliche Behandlung zur Linderung der Beschwerden.

### MATERIAL UND METHODEN

### Patienten und Kontrollgruppenselektion

Die Studienpopulation setzt sich aus zwei Patientengruppen und einer gesunden Kontrollgruppe (HD = healthy donors) zusammen. Bei den Untersuchungsgruppen handelt es sich entweder um Arthrose- oder rheumatoide Arthritis (RA)-Patienten. Die Einschlusskriterien für die Arthrose-Gruppe sind eine klinisch gesicherte Osteoarthritis (OA) der großen Gelenke. Ausschlusskriterien sind Hinweise auf eine rheumatoide Gelenkerkrankung oder andere autoimmune oder maligne Grunderkrankungen. Einschlusskriterien für die RA-Gruppe sind eine gesicherte RA entsprechend der ACR/EULAR-Klassifikationskriterien. Ausschlusskriterien sind eine Arthrose oder andere autoimmune oder maligne Grunderkrankungen. Zum Einschluss in die Kontrollgruppe müssen die Probanden artikulär symptomfrei sein. Ausschlusskriterien sind diagnostische Hinweise auf eine Arthrose, RA, autoimmune oder maligne Grunderkrankungen. Untersucht wurde das Serum von 10 Arthrose-Patienten, 10 RA-Patienten und 10 gesunden Kontrollen. Alle Proben wurden von unserem Kooperationspartner Prof. Pongratz, Rheumatologie der Universitätsklinik Düsseldorf, zur Verfügung gestellt.

### Klonierung, rekombinante Expression und Proteinaufreinigung

Das Gen von humanem CLEC3A wurde in einen modifizierten pCEP-Pu-Vektor kloniert und in HEK-293 EBNA Zellen transfiziert. Das rekombinante Protein wurde affinitätschromatographisch aus dem Überstand der Zellen aufgereinigt. Humanes Kollagen II wurde aus humanem Knorpel aufgereinigt (PMID: 6439184). Rekombinantes humanes TSP-4 (R&D) und rekombinantes humanes COMP (R&D) wurden beim jeweiligen Hersteller bestellt.

### SDS-Polyacrylamid-Gelelektrophorese (SDS-PAGE) und Immunblot

Zum Nachweis von Autoantikörpern im Serum der Probanden wurden die Matrixproteine mittels SDS-PAGE aufgetrennt und mittels Immunblot nachgewiesen. Hierfür wurden die Proteine (je 1 µg/Vertiefung) nach Durchführung der SDS-PAGE (4-12% Bis-Tris-Gels: 12 Vertiefungen, 1 mm Dicke, MOPS Puffer, 200 V für 50 min) auf eine PVDF-Membran transferiert (0,45 µm, Invitrogen), freie Bindungsstellen mit 5% Milchpulver und 1% bovinem Serumalbumin in TBS-T (Tris-gepufferte Salzlösung, 0,1% Tween) abgesättigt, nacheinander mit 50 µl Patientenserum in 10 ml Blockierungs-Lösung, über Nacht bei 4°C) und einem HRP (Meerrettich-Peroxidase)-konjugiertem anti-human-IgG-Antikörper (SantaCruz, 1:200.000 in Blocking für 1 h Raumtemperatur) inkubiert. Als Referenz wurde Serum einer gesunden Person verwendet. Die Konzentration von IgG im Serum wurde bestimmt und es wurde jeweils 0,3, 3 oder 30 ng IgG pro Referenzspur auf das SDS-PAGE aufgetragen. Signale wurden nach Inkubation mit ECL Plus (Amersham Pharmacia Biotech) mit dem ChemiDoc XRS+ (BioRad) Westernblot-Imager detektiert und die Bildsequenzen mit der ImageLab (BioRad)-Software ausgewertet.

### ERGEBNISSE UND DISKUSSION

**Nachweis von TSP-4-, CLEC3A-, COMP- und Kollagen II-Autoantikörper zur Diagnostik einer Arthrose** - In unsere Studie haben wir mittels SDS-PAGE und Immunblot das Serum von 10 Arthrose (OA)-Patienten, 10 Rheumatoide-Arthritis (RA)-Patienten und 10 gesunden Probanden (Healthy Donors, HD) auf IgG-Isotyp-Antikörper gegen TSP-4, CLEC3A, COMP und Kollagen II untersucht (Tabelle 1). Der Nachweis von TSP4-, CLEC3A-, COMP- und Kollagen-II-Autoantikörpern im Blut und/oder in Synovialflüssigkeit kann zur Diagnostik, speziell auch zu einer frühen Diagnostik, einer Arthrose oder einer aktivierten Arthrose sowie zur Stadiendiagnose, Verlaufs- und Therapiekontrolle einer Arthrose genutzt werden (Tabelle 1).

**Tabelle 1a: Anzahl der positiven Antikörper-Reaktivitäten gegen die verschiedenen Matrixproteine im Vergleich von OA und HD. Häufigkeiten wurden jeweils in Relation zur Gruppenstärke angegeben. Signifikante Unterschiede sind mit * markiert. (Quelle: http://www.socscistatistics.com/tests/fisher/Default2.aspx).**

| Antikör-per gegen: | OA | HD | positiver Vorhersagewert | negativer Vorhersagewert | Sensitivität | Spezifität | p-Wert |
|---|---|---|---|---|---|---|---|
| TSP-4 * | 5/10 | 0/10 | 1 | 0,67 | 0,5 | 1 | 0,0325 |
| COMP | 4/10 | 1/10 | 0,8 | 0,67 | 0,4 | 0,9 | 0,303 |
| CLEC3A | 2/10 | 0/10 | 1 | 0,56 | 0,2 | 1 | 0,474 |
| Collagen II | 0/10 | 7/10 | 0 | 0,23 | 0 | 0,3 | 0,003 |

**Tabelle 1b: Individuelle Antikörperintensitäten gegen verschiedene Antigene. Patienten 1 bis 10 repräsentieren die OA-Gruppe, Patienten 11 bis 20 die gesunde Kontrollgruppe. Die Mittelwerte und Standardabweichungen wurden für die entsprechende Gruppe berechnet, in denen für das respektive Antigen ≥ zwei Werte vorlagen. Signale des Immunblots wurden densitometrisch ausgewertet und im Verhältnis zur zugehörigen 0,3 ng-IgG-Referenzbande (=1) quantifiziert (x-fach IgG) oder im Verhältnis zum Gruppenmittelwert (x-fach MW) angegeben. Wenn in einem Kästchen keine Werte eingetragen wurden, konnte im Immunblot keine Bande nachgewiesen werden. MW = Mittelwert, StdAbw = Standardabweichung.**

| | TSP-4 x-fach IgG-Referenz | TSP-4 x-fach MW | COMP x-fach IgG-Referenz | COMP x-fach MW | CLEC3A x-fach IgG-Referenz | CLEC3A x-fach MW | Kollagen II x-fach IgG-Referenz | Kollagen II x-fach MW |
|---|---|---|---|---|---|---|---|---|
| MW | 0,41 | 1 | 0,27 | 1 | 0,27 | 1 | 0,40 | 1 |
| MW -/+ 2x StdAbw | -0,32 bis 1,13 | - | -0,22 bis 0,77 | - | 0,12 bis 0,42 | - | 0,27 bis 0,53 | - |
| Patient 1 | 0,96 | 2,36 | | | | | | |
| Patient 2 | | | 0,15 | 0,55 | 0,19 | 0,72 | | |
| Patient 3 | 0,15 | 0,37 | | | | | | |
| Patient 4 | 0,09 | 0,22 | | | | | | |
| Patient 5 | 0,72 | 1,77 | | | | | | |
| Patient 6 | | | 0,2 | 0,73 | | | | |
| Patient 7 | | | 0,05 | 0,18 | | | | |
| Patient 8 | | | | | | | | |
| Patient 9 | 0,11 | 0,27 | 0,69 | 2,53 | | | | |
| Patient 10 | | | | | 0,34 | 1,28 | | |
| Patient 11 | | | | | | | 0,33 | 0,83 |
| Patient 12 | | | | | | | 0,42 | 1,05 |
| Patient 13 | | | | | | | | |
| Patient 14 | | | | | | | | |
| Patient 15 | | | | | | | 0,53 | 1,33 |
| Patient 16 | | | | | | | 0,35 | 0,88 |
| Patient 17 | | | | | | | 0,34 | 0,85 |
| Patient 18 | | | | | | | 0,39 | 0,98 |
| Patient 19 | | | | | | | 0,44 | 1,10 |
| Patient 20 | | | | | | | | |

Durch die Kombination der Antikörper kann die diagnostische Aussagekraft noch deutlich erhöht werden (Tabelle 2).

**Tabelle 2: Anzahl positiver Antikörper-Reaktivitäten, kumuliert gegen mehrere Proteine in den Gruppen OA und HD. Häufigkeiten wurden jeweils in Relation zur Gruppenstärke angegeben. Für die kumulativen Vergleiche sind die Unterschiede in der Verteilung der absoluten Häufigkeiten auf die zwei Gruppen hochsignifikant. Kollagen II-Autoantikörper konnten nur in der HD-Gruppe nachgewiesen werden. OA-Patienten zeigten keine Kollagen II-Autoantikörper. Der fehlende Nachweis von Kollagen II-Autoantikörpern dient daher als positives Ergebnis für OA.**

| Gruppe Antikörper | OA | HD | positiver Vorhersagewert | negativer Vorhersagewert | Sensitivität | Spezifität | p-Wert |
|---|---|---|---|---|---|---|---|
| TSP-4 | 5/10 | 0/10 | 1 | 0,67 | 0,5 | 1 | 0,033 |
| TSP-4 / COMP | 8/10 | 1/10 | 0,89 | 0,82 | 0,8 | 0,9 | 0,005 |
| TSP-4 / COMP / CLEC3A | 9/10 | 1/10 | 0,9 | 0,9 | 0,9 | 0,9 | 0,001 |
| TSP-4 / COMP / CLEC3A / Collagen II | 10/10 | 3/10 | 0,77 | 1,0 | 1,0 | 0,7 | 0,003 |

**Nachweis von TSP-4-, CLEC3A- und COMP-Antikörpern zur Therapieauswahl bei Arthrose** - In verschiedenen Studien wurde gezeigt, dass es durch die Immunisierung von Tieren mit Kollagen II (CIA, Collagen II-induced arthritis) oder COMP (COMPIA) zu einer schweren, chronischen Arthritis bei den Tieren kommt. Antikörper gegen Matrixproteine haben in diesen Tiermodellen (der RA) eine pathophysiologische Bedeutung. Dies zeigt, dass auch humane Autoantikörper gegen Matrixproteine eine pathophysiologische Wirkung entfalten können und deshalb Patienten mit Autoantikörpern gegen Matrixproteine einer immunsuppressiven Therapie zugänglich sind. Dabei scheint ein wichtiges Kriterium für die Pathogenität die Anzahl von verschiedenen Autoantikörpern und/oder die Autoantikörperkonzentration (Autoantikörpertiter) im Blut zu sein. In unserer Studie liegen bei zwei OA-Patienten mehrere Autoantikörper vor und auch die Antikörperintensitäten gegen die verschiedenen Antigene sind unterschiedlich.

**Nachweis von anti-TSP-4- und anti-COMP-Antikörpern bei rheumatoider Arthritis** - In unserem Studienkollektiv fanden wir bei drei RA-Patienten um den Faktor 30-fach bis 130-fach höhere Konzentrationen von TSP-4- und COMP-Autoantikörpern (RA-Patient Nr. 21, 22, 29), verglichen mit den Intensitäten der anderen RA-Patienten (Tabelle 3).

**Tabelle 3: Mittelwerte der Antikörperintensitäten in der RA-Gruppe. Zur Mittelwertberechnung wurden Patienten 21, 22, 29 ausgeschlossen, da sie statistisch gesehen (Ausreißertest nach Grubbs) signifikante Ausreißer waren. MW = Mittelwert, StdAbw = Standardabweichung. Signale des Immunblots wurden densitometrisch ausgewertet und im Verhältnis zur zugehörigen 0,3 ng-IgG-Referenzbande (=1) quantifiziert (x-fach IgG) oder im Verhältnis zum Gruppenmittelwert (x-fach MW) angegeben.**

| | TSP-4 x-fach IgG-Referenz | TSP-4 x-fach MW] | COMP x-fach IgG-Referenz | COMP x-fach MW] |
|---|---|---|---|---|
| MW | 0,95 | 1 | 0,63 | 1 |
| MW -/+ 2x StdAbw | -0,60 bis 2,50 | - | -0,05 bis 1,32 | - |
| **Patient 21** | 1,84 | 1,93 | **82,9** | **130,67** |
| **Patient 22** | **30,7** | **32,27** | 0,9 | 1,42 |
| Patient 23 | 1,3 | 1,37 | 0,81 | 1,28 |
| Patient 24 | 0,81 | 0,85 | 1,29 | 2,03 |
| Patient 25 | 0,52 | 0,55 | 0,45 | 0,71 |
| Patient 26 | Keine Bande | Keine Bande | 0,13 | 0,20 |
| Patient 27 | 0,19 | 0,20 | 0,28 | 0,44 |
| Patient 28 | 0,57 | 0,60 | 0,45 | 0,71 |
| **Patient 29** | **51,8** | **54,45** | 0,53 | 0,84 |
| Patient 30 | 2,38 | 2,50 | 0,87 | 1,37 |

### REFERENZEN

Aletaha D, Neogi T, Silman AJ, et al 2010 Rheumatoid arthritis classification criteria: an American College of Rheumatology/European League Against Rheumatism collaborative initiative Annals of the Rheumatic Diseases 2010, 69, 1580-1588
Deane KD., Curr Rheumatol Rep. 2014, 16, 419
Jescke A, et al. Deficiency of thrombospondin-4 in mice does not affect skeletal growth or bone mass acquisition, but causes a transient reduction of articular cartilage thickness. PLOS ONE 2015; DOI 10.1371
Karlin S. und Altschul S.F., PNAS, 1990, 87, 2264-2268
Karlin S. und Altschul S.F., PNAS, 1993, 90, 5873-5877
Lawler J, McHenry K, Duquette M, Derick L (Mar 1995). "Characterization of human thrombospondin-4". J Biol Chem. 270 (6): 2809-14. doi:10.1074/jbc.270.6.2809. PMID 7852353
Needleman S.B. und Wunsch C. D., J Mol Biol, 1970, 48, 443-453
Orlandi et al., 1989, Proc. Natl. Acad. Sci. USA 86: 3833-3837; und Winter-und Milstein, 1991, Nature 349: 293-299
Pearson W.R. und Lipman D. J., PNAS, 1988, 85, 2444-2448
Smith T.F. und Waterman M.S., Add APL Math, 1981, 2, 482-489
Stoll ML, Li QZ, Zhou J, Punaro M, Olsen NJ. Elevated IgG autoantibody production in oligoarticular juvenile idiopathic arthritis may predict a refractory course. Clin Exp Rheumatol. 2011; 29: 736-742.
   1. Newton, G., et al., Characterization of human and mouse cartilage oligomeric matrix protein. Genomics, 1994. 24(3): p. 435-9.
   2. DiCesare, P.E., et al., Cartilage oligomeric matrix protein: isolation and characterization from human articular cartilage. J Orthop Res, 1995. 13(3): p. 422-8.
   3. Acharya, C., et al., Cartilage oligomeric matrix protein and its binding partners in the cartilage extracellular matrix: interaction, regulation and role in chondrogenesis. Matrix Biol, 2014. 37: p. 102-11.
   4. Halasz, K., et al., COMP acts as a catalyst in collagen fibrillogenesis. J Biol Chem, 2007. 282(43): p. 31166-73.
   5. Haudenschild, D.R., et al., Enhanced activity of transforming growth factor beta1 (TGF-beta1) bound to cartilage oligomeric matrix protein. J Biol Chem, 2011. 286(50): p. 43250-8.
   6. Ishida, K., et al., Cartilage oligomeric matrix protein enhances osteogenesis by directly binding and activating bone morphogenetic protein-2. Bone, 2013. 55(1): p. 23-35.
   7. Rosenberg, K., et al., Cartilage oligomeric matrix protein shows high affinity zinc-dependent interaction with triple helical collagen. J Biol Chem, 1998. 273(32): p. 20397-403.
   8. Kleerekoper, Q., J.T. Hecht, and J.A. Putkey, Disease-causing mutations in cartilage oligomeric matrix protein cause an unstructured Ca2+ binding domain. J Biol Chem, 2002. 277(12): p. 10581-9.
   9. Kvansakul, M., J.C. Adams, and E. Hohenester, Structure of a thrombospondin C-terminal fragment reveals a novel calcium core in the type 3 repeats. EMBO J, 2004. 23(6): p. 1223-33.
   10. Lau, D., et al., The cartilage-specific lectin C-type lectin domain family 3 member A (CLEC3A) enhances tissue plasminogen activator-mediated plasminogen activation. J Biol Chem, 2017.
   11. Karlsson, C., et al., Genome-wide expression profiling reveals new candidate genes associated with osteoarthritis. Osteoarthritis Cartilage, 2010. 18(4): p. 581-92.
   12. Tsunezumi, J., S. Higashi, and K. Miyazaki, Matrilysin (MMP-7) cleaves C-type lectin domain family 3 member A (CLEC3A) on tumor cell surface and modulates its cell adhesion activity. J Cell Biochem, 2009. 106(4): p. 693-702.
   13. Neame, P.J., H. Tapp, and D.R. Grimm, The cartilage-derived, C-type lectin (CLECSF1): structure of the gene and chromosomal location. Biochim Biophys Acta, 1999. 1446(3): p. 193-202.
   14. Vogel, K.G., M. Paulsson, and D. Heinegard, Specific inhibition of type I and type II collagen fibrillogenesis by the small proteoglycan of tendon. Biochem J, 1984. 223(3): p. 587-97.
   15. Altman, R.D. Classification of disease: osteoarthritis. in Seminars in arthritis and rheumatism. 1991. Elsevier.
   16. Altman, R., et al., Development of criteria for the classification and reporting of osteoarthritis. Classification of osteoarthritis of the knee. Diagnostic and Therapeutic Criteria Committee of the American Rheumatism Association. Arthritis Rheum, 1986. 29(8): p. 1039-49.
   17. Altman, R., et al., The American College of Rheumatology criteria for the classification and reporting of osteoarthritis of the hand. Arthritis Rheum, 1990. 33(11): p. 1601-10.

## Patentansprüche

1. Verfahren zur Diagnose von Arthrose oder des Risikos, Arthrose zu entwickeln, bei einem Subjekt, wobei das Subjekt keine rheumatoide Arthritis aufweist, umfassend das Nachweisen eines Autoantikörpers, der mit Arthrose assoziiert ist, in einer von dem Subjekt stammenden Probe.

2. Verfahren nach Anspruch 1, wobei das Verfahren das Ausschließen von rheumatoider Arthritis bei dem Subjekt umfasst.

3. Verfahren nach Anspruch 1 oder 2, weiterhin umfassend das Ausschließen des Vorliegens eines Autoantikörpers gegen Kollagen II oder ein Degradationsprodukt oder ein Fragment davon,
bevorzugt wobei das Verfahren das Nachweisen eines Autoantikörpers gegen Kollagen II oder ein Degradationsprodukt oder ein Fragment davon umfasst, stärker bevorzugt wobei das Verfahren das Nachweisen eines Autoantikörpers gegen Kollagen II oder ein Degradationsprodukt oder ein Fragment davon mit einem Nachweisagens zum Nachweisen eines Autoantikörpers gegen Kollagen II oder ein Degradationsprodukt oder ein Fragment davon umfasst.

4. Verfahren nach einem der Ansprüche 1 bis 3, wobei das Verfahren das Nachweisen von einem Autoantikörper gegen Thrombospondin-4 (TSP-4) oder ein Degradationsprodukt oder ein Fragment davon oder das Nachweisen von einem Autoantikörper gegen COMP oder ein Degradationsprodukt oder ein Fragment davon umfasst.

5. Verfahren nach einem der Ansprüche 1 bis 4, wobei der Verlauf von Arthrose überwacht wird, umfassend den Nachweis des Autoantikörpers zu verschiedenen Zeitpunkten; oder
wobei das Stadium von Arthrose diagnostiziert wird.

6. Verfahren zur Diagnose einer degenerativen Erkrankung des Skelettsystems oder des Risikos, eine degenerative Erkrankung des Skelettsystems zu entwickeln, bei einem Subjekt, umfassend das Nachweisen von einem Autoantikörper gegen Thrombospondin-4 (TSP-4) oder ein Degradationsprodukt oder ein Fragment davon oder das Nachweisen von einem Autoantikörper gegen COMP oder ein Degradationsprodukt oder ein Fragment davon in einer von dem Subjekt stammenden Probe,
bevorzugt wobei die degenerative Erkrankung des Skelettsystems rheumatoide Arthritis oder Arthrose umfasst.

7. Verfahren nach einem der Ansprüche 1 bis 6, wobei das Verfahren das Nachweisen von mindestens zwei Autoantikörpern umfasst;
bevorzugt wobei das Verfahren das Nachweisen von einem Autoantikörper gegen Thrombospondin-4 (TSP-4) oder ein Degradationsprodukt oder ein Fragment davon und das Nachweisen von einem Autoantikörper gegen COMP oder ein Degradationsprodukt oder ein Fragment davon umfasst; oder
bevorzugt wobei das Verfahren das Nachweisen von einem Autoantikörper gegen Thrombospondin-4 (TSP-4) oder ein Degradationsprodukt oder ein Fragment davon und das Nachweisen von einem Autoantikörper gegen CLEC3A oder ein Degradationsprodukt oder ein Fragment davon umfasst;
stärker bevorzugt wobei das Verfahren das Nachweisen von einem Autoantikörper gegen Thrombospondin-4 (TSP-4) oder ein Degradationsprodukt oder ein Fragment davon, das Nachweisen von einem Autoantikörper gegen COMP oder ein Degradationsprodukt oder ein Fragment davon und das Nachweisen von einem Autoantikörper gegen CLEC3A oder ein Degradationsprodukt oder ein Fragment davon umfasst.

8. Verfahren nach einem der Ansprüche 1 bis 7, wobei der Autoantikörper gegen ein Neoepitop eines Degradationsprodukts eines Proteins, gegen das der Autoantikörper gerichtet ist, gerichtet ist.

9. Verfahren nach einem der Ansprüche 1 bis 8, wobei das Nachweisen mit einem Nachweisagens erfolgt,
bevorzugt wobei das Nachweisagens fähig ist, an die Antigenbindungsregion des Autoantikörpers zu binden,
stärker bevorzugt wobei das Nachweisagens TSP-4 oder ein Degradationsprodukt oder ein Fragment davon, COMP oder ein Degradationsprodukt oder ein Fragment davon und/oder CLEC3A oder ein Degradationsprodukt oder ein Fragment davon ist.

10. Verfahren zur Diagnose von Arthrose oder des Risikos, Arthrose zu entwickeln, bei einem Subjekt, umfassend das Ausschließen des Vorliegens eines Autoantikörpers gegen Kollagen II oder ein Degradationsprodukt oder ein Fragment davon in einer von dem Subjekt stammenden Probe;
bevorzugt wobei das Verfahren das Nachweisen eines Autoantikörpers gegen Kollagen II oder ein Degradationsprodukt oder ein Fragment davon umfasst;
stärker bevorzugt wobei das Verfahren das Nachweisen eines Autoantikörpers gegen Kollagen II oder ein Degradationsprodukt oder ein Fragment davon mit einem Nachweisagens zum Nachweisen eines Autoantikörpers gegen Kollagen II oder ein Degradationsprodukt oder ein Fragment davon umfasst.

11. Verfahren nach Anspruch 10, wobei die Arthrose zusätzlich durch herkömmliche Methoden diagnostiziert wird.

12. Verfahren nach einem der Ansprüche 1 bis 11, wobei die Probe Körperflüssigkeit, bevorzugt Blut, Serum, Blutplasma, Synovialflüssigkeit, Urin, Muskel- oder Knorpelgewebe, Synovialmembran oder Sehne ist.

13. Kit zur Diagnose von Arthrose oder des Risikos, Arthrose zu entwickeln, bei einem Subjekt, wobei das Subjekt keine rheumatoide Arthritis aufweist, umfassend ein Nachweisagens zum Nachweisen von einem Autoantikörper, der mit Arthrose assoziiert ist,
bevorzugt wobei das Nachweisagens ein Nachweisagens zum Nachweisen von einem Autoantikörper gegen TSP-4 oder ein Degradationsprodukt oder ein Fragment davon oder ein Nachweisagens zum Nachweisen von einem Autoantikörper gegen COMP oder ein Degradationsprodukt oder ein Fragment davon umfasst; oder
Kit zur Diagnose einer degenerativen Erkrankung des Skelettsystems oder des Risikos, eine degenerative Erkrankung des Skelettsystems zu entwickeln, bei einem Subjekt, umfassend ein Nachweisagens zum Nachweisen von einem Autoantikörper gegen Thrombospondin-4 (TSP-4) oder ein Degradationsprodukt oder ein Fragment davon oder ein Nachweisagens zum Nachweisen von einem Autoantikörper gegen COMP oder ein Degradationsprodukt oder ein Fragment davon umfasst,
bevorzugt wobei die degenerative Erkrankung des Skelettsystems rheumatoide Arthritis oder Arthrose umfasst.

14. Kit nach Anspruch 13, wobei das Nachweisagens mindestens zwei Nachweisagentien zum Nachweisen von mindestens zwei Autoantikörpern umfasst,
bevorzugt wobei das Nachweisagens ein Nachweisagens zum Nachweisen von einem Autoantikörper gegen Thrombospondin-4 (TSP-4) oder ein Degradationsprodukt oder ein Fragment davon und ein Nachweisagens das Nachweisen von einem Autoantikörper gegen COMP oder ein Degradationsprodukt oder ein Fragment davon umfasst; oder
bevorzugt wobei das Nachweisagens ein Nachweisagens zum Nachweisen von einem Autoantikörper gegen Thrombospondin-4 (TSP-4) oder ein Degradationsprodukt oder ein Fragment davon und ein Nachweisagens das Nachweisen von einem Autoantikörper gegen CLEC3A oder ein Degradationsprodukt oder ein Fragment davon umfasst;
stärker bevorzugt wobei das Nachweisagens ein Nachweisagens zum Nachweisen von einem Autoantikörper gegen Thrombospondin-4 (TSP-4) oder ein Degradationsprodukt oder ein Fragment davon, ein Nachweisagens zum Nachweisen von einem Autoantikörper gegen COMP oder ein Degradationsprodukt oder ein Fragment davon und ein Nachweisagens zum Nachweisen von einem Autoantikörper gegen CLEC3A oder ein Degradationsprodukt oder ein Fragment davon umfasst.

15. Kit nach Anspruch 13 oder 14, wobei der Kit weiterhin ein Nachweisagens zum Nachweisen von einem Autoantikörper gegen Kollagen II oder ein Degradationsprodukt oder ein Fragment davon umfasst.

16. Verwendung des Kits nach einem der Ansprüche 13 bis 15 zur Diagnose von Arthrose oder des Risikos, Arthrose zu entwickeln, bei einem Subjekt, wobei das Subjekt keine rheumatoide Arthritis aufweist; oder
zur Diagnose von einer degenerativen Erkrankung des Skelettsystems oder des Risikos, eine degenerative Erkrankung des Skelettsystems zu entwickeln, bei einem Subjekt,
bevorzugt wobei die degenerative Erkrankung rheumatoide Arthritis oder Arthrose umfasst.

17. Wirkstoff zur Verwendung zur Behandlung oder Vorbeugung einer autoimmun-assoziierten Arthrose bei einem Subjekt, bevorzugt wobei der Wirkstoff ein Antikörper gegen B-Zellen, stärker bevorzugt Rituximab, oder ein Wnt-Signalisierungs-Inhibitor, stärker bevorzugt Fluoxetin oder SM04690, ist.

18. Verfahren nach einem der Ansprüche 1 bis 13, wobei das Verfahren zur Therapieauswahl eingesetzt wird.
